# EUROPEAN PATENT APPLICATION

(11) **EP 4 825 389 A1**
(43) Date of publication of application: **21.10.2026**
(21) Application number: 25171210.5
(22) Date of filing: 17.04.2025
(51) Int. Cl.: A61K 38/16, A61K 39/02, C07K 14/315

(54) **METHOD FOR PURIFYING A RECOMBINANT FUSION PROTEIN**

(71) Applicant: MinervaX ApS, 2000 Frederiksberg (DK)
(72) Inventor: BOMHOLT, Julie, 2920 CHARLOTTENLUND (DK); KANTSØ, Bjørn, 4300 HOLBÆK (DK)
(74) Representative: Öberg & Sjöberg AB

(57) **Abstract**

The present document is directed to a method for purification of a recombinant fusion protein comprising the N-terminal of two group B *Streptococcus* surface proteins. The method comprises a series of chromatography purification steps optimised to enable large scale production of the recombinant fusion proteins with quality sufficient for therapeutic use and in high yields.

## Description

### TECHNICAL FIELD

The technology proposed herein relates to the fields of microbiology and vaccine technology and concerns the production of a recombinant fusion protein capable of conferring immunity to group B *Streptococcus* infections. More particularly, the technology proposed herein relates to a chromatographic method for the purification of such recombinant fusion proteins, wherein the method involves several, carefully selected, different chromatographic steps to enable the production of therapeutic grade recombinant fusion protein in high yields.

### BACKGROUND

Group B *Streptococcus* (*Streptococcus agalactiae*) (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economic importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (la, lb, and II-VIII) based on the structure of the polysaccharide capsule. The four "classical" serotypes la, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

The document Lindahl et al., Nonimmunodominant Regions Are Effective As Building Blocks In A Streptococcal Fusion Protein Vaccine, Cell Host & Microbe 2, 427-434, December 2007, discloses a fusion protein comprising N-terminal regions of the group B *Streptococcus* surface proteins Rib and AlpC.

Similarly, WO 2008127179 describes a fusion protein comprising at least one first N-terminal region fragment of a group B *Streptococcus* surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B S *Streptococcus* surface proteins derive from different group B *Streptococcus* strains, and wherein the fusion protein is capable of eliciting protective immunity against group B *Streptococcus.*

Despite the advances in the progress towards a vaccine suitable for prevention of GBS disease, there is still a need for further methods and vaccines for prevention and treatment of GBS infections. Also, a successful vaccine strategy relies on the ability to efficiently being able to produce sufficient amounts of vaccine of good quality.

Accordingly, it is a primary objective of the technology proposed herein to provide an improved method for the purification of recombinant fusion proteins, in particular recombinant fusion proteins that are to be used for immunization.

Another objective of the technology proposed herein is to provide recombinant fusion proteins that are suitable to use for immunization, i.e. recombinant fusion proteins of sufficiently high purity.

Yet another objection of the technology proposed herein is to provide a purification method for recombinant fusion proteins that allow sufficiently high yields of the proteins to be obtained.

The means of accomplishing each of the above objectives as well as others will become apparent from the description which follows hereafter.

### SUMMARY

A first aspect of the technology proposed herein is directed to a chromatographic method for purifying a homogenate comprising a recombinant fusion protein comprising or consisting of a first amino acid sequence having at least 90% sequence identity with the amino acid sequence of the N-terminal region of a first group B *Streptococcus* surface protein, said first amino acid sequence being fused to, optionally via linker, a second amino acid sequence having at least 90% sequence identity with the amino acid sequence of the N-terminal region of a second group B *Streptococcus* surface protein, said method comprising the steps of:
i) passing the homogenate through an anion exchange column configured to retain charged contaminants,
ii) optionally passing the homogenate obtained in step i) through a hydrophobic interaction column configured to retain hydrophobic contaminants,
iii) passing the homogenate obtained in step i) or ii) through a mixed mode column to polish the homogenate,
iv) exchanging the buffer of the homogenate obtained in step iii) to a final buffer using a desalting column, and
v) passing the homogenate obtained in step iv) through a membrane filter, to obtain the purified recombinant fusion protein in the final buffer.

A second aspect of the technology proposed herein, is directed to a system comprising or consisting of:
i) an anion exchange column configured to retain charged contaminants,
ii) optionally a hydrophobic interaction column configured to retain hydrophobic contaminants,
iii) a mixed mode column configured to polish the homogenate,
iv) a desalting column for buffer exchange, and
v) a membrane filter, such as a 0.2 µm filter.

Such a system is adapted to perform the method according to the first aspect of the technology proposed herein.

Other features and advantages of the technology proposed herein will be apparent from the following detailed description, drawings, examples, and from the claims.

### DEFINITIONS AND ABBREVIATIONS

AIEX: Anion exchange chromatography
CIEX: Cation exchange chromatography
DSP: Downstream process
GBS: Group B Streptococcus
GMP: Good Manufacturing Practice
HA: Hydroxyapatite (chromatography)
HCP: Host cell proteins (e.g. E. coli proteins)
HIC: Hydrophobic interaction chromatography
MMC: Multi-modal chromatography/mixed mode chromatography
MWCO: Molecular weight cut-off
SEC: Size exclusion chromatography
TFF: Tangential Flow Filtration
GBS-NN, NN-GBS, NN_GBS and the like are used interchangeably herein.
GBS-NN2, NN2-GBS, NN2_GBS and the like are used interchangeably herein.

### DESCRIPTION OF DRAWINGS

Fig. 1. Shows an SDS-PAGE gel of the cell extract, the anion exchange product and the hydrophobic interaction chromatography product. Lane 1: See blue plus 2, lane 2: Cell extract, diluted 10-fold, lane 3: Anion exchange product, diluted 10-fold, lane 4: HIC product, lane 5: See blue plus 2.
Fig. 2. Shows a chromatogram from anion exchange chromatography on a 20-ml DEAE Sepharose FF column (HiPrep DEAE).
Fig. 3. Shows an SDS PAGE gel with Coomassie blue staining showing different fractions from anion exchange chromatography on a 20-ml DEAE Sepharose FF column (HiPrep DEAE). M: MW marker, Mark 12 (Invitrogen), Ld: E. coli extract, 2 µL loaded, A3-D10: Collected fractions (7 ml), 5 uL loaded. Arrow indicates target protein.
Fig. 4. Shows a chromatogram from Hydrophobic interaction chromatography on 20-ml Phenyl Sepharose FF low sub column.
Fig. 5. Shows an SDS PAGE gel with Coomassie blue staining showing different fractions from hydrophobic interaction chromatography. M: MW marker, Mark 12 (Invitrogen), Ld: Pool from step one, 1 uL loaded, C7-D5: Collected fractions (7 ml), 5 uL loaded. Fractions C10-D8 were pooled.
Fig. 6. Shows a chromatogram of GBS-NN BX1015300 small scale AIEX run. The pooled fractions are indicated by Fraction@11,HIST.
Fig. 7. Shows a chromatogram of GBS-NN BX1015300 small scale HIC run. The pooled fractions are indicated by Fraction@11,HIST.
Fig. 8. Shows a chromatograms of Capto MMC Run 2. Pooled fractions are indicated by Fraction@11,HIST.
Fig. 9. Shows a chromatogram of Capto Adhere Run 1. Pooled fractions are indicated by Fraction@11,HIST.
Fig. 10. Shows a chromatogram of Ceramic HA Run 1. Pooled fractions are indicated by Fraction@11,HIST.
Fig. 11. Shows a chromatogram of BX1015703 AlEX XK16 run (GBS-NN2). The pooled fractions are indicated by Fraction@11,HIST.
Fig. 12. Shows a chromatogram of BX1015703 HIC XK16 run (GBS-NN2). The pooled fractions are indicated by Fraction@11,HIST.
Fig. 13. Shows a chromatogram of GBS-NN2 Capto Adhere Run 1. The pooled fractions are indicated by Fraction@11,HIST.
Fig. 14. Shows an overlay comparison of GBS-NN and GBS-NN2 Capto Adhere runs.
Fig. 15. Shows a chromatogram of GBS-NN Capto Adhere Run 2. Elution using 25/60/100 % buffer B.
Fig. 16. Shows a chromatogram of GBS-NN Capto Adhere Run 3. Elution using 20/65/100 % buffer B.
Fig. 17. Shows a chromatogram of GBS-NN2 Capto Adhere Run 2. Elution using 20/65/100 % buffer B.
Fig. 18. Shows an overlay comparison of GBS-NN Capto Adhere Run 3 (blue) and GBS-NN2 Capto Adhere Run 2 (orange), when using elution with 20/65/100 % buffer B.
Fig. 19. Shows a chromatogram of GBS-NN Capto Adhere Run 4. 20 cm column, elution using 20/70/100 % buffer B.
Fig. 20. Shows a chromatogram of GBS-NN Capto Adhere Run 5. Elution using 17/55/100 % buffer B.
Fig. 21. Shows a chromatogram of GBS-NN Capto Adhere Run 6. Flow rate 300 cm/h, elution using 17/55/100 % buffer B.
Fig. 22. Shows a chromatogram of GBS-NN Capto Adhere Capacity run.
Fig. 23. Shows a chromatogram of GBS-NN Capto Adhere Run 7. Load 95 % of maximum load (19 mg/mL resin).
Fig. 24. Shows a chromatogram of GBS-NN Capto Adhere Run 8. Load 75 % of maximum load (15 mg/ml resin). The pooled fractions are indicated by Fraction@11,HIST.
Fig. 25. Shows a chromatogram of GBS-NN Capto Adhere Run 9. Load 65 % of maximum load (13 mg/ml resin). The pooled fractions are indicated by Fraction@11,HIST.
Fig. 26. Shows a chromatogram of GBS-NN Capto Adhere Run 10. Load 80 % of maximum load (16 mg/ml resin). The pooled fractions are indicated by Fraction@11,HIST.
Fig. 27. Shows a process value graphs of GBS-NN TFF runs 1-3.
Fig. 28. Shows a process value graph of GBS-NN TFF run 4.
Fig. 29. Shows a chromatograms of Desalting Run 1 (top), Run 2 (middle) and a comparison of the two runs (bottom). The pooled fractions are indicated by Fraction@11,HIST.
Fig. 30. Shows a chromatogram of Desalting Run 3 (top) and comparison of Run 1 and Run 3 (same load volume, different load concentration, different flow rate). Sample elution properties are nearly identical between runs 1 and 3. The pooled fractions are indicated by Fraction@11,HIST.
Fig. 31. Shows a chromatogram of Desalting Run 4. The pooled fractions are indicated by Fraction@11,HIST.
Fig. 32. Shows an overlay comparison of Desalting run chromatograms of runs 1, 2 and 4 (20%, 30% and 25% load, respectively). Bed height was 10 cm in runs 1 and 2, and 20 cm in run 4.
Fig. 33. Shows an overview of the purification method.

### DETAILED DESCRIPTION

When producing proteins for medical use, such as for use in immunisation, it is of outmost importance that the resulting proteins are of high quality and purity. Further, the production method must enable a high yield of product to be obtained at a reasonable cost.

According to the technology proposed herein, there is provided a method for producing a recombinant fusion protein that enables a high yield of high-quality product to be obtained. The method is based on different chromatographic purification steps, the types and order carefully being selected to obtain the best results. Surprisingly, standard method steps commonly used for protein purification were found to not be optimal and unexpected method steps had to be selected to achieve a good result.

According to a first aspect of the technology proposed herein there is provided a chromatographic method for purifying a homogenate comprising a recombinant fusion protein. The method comprises the steps of:
i) passing a homogenate comprising the recombinant fusion protein through an anion exchange column configured to retain charged contaminants,
ii) optionally passing the homogenate obtained in step i) through a hydrophobic interaction column configured to retain hydrophobic contaminants,
iii) passing the homogenate obtained in step i) or ii) through a mixed mode column to polish the homogenate,
iv) exchanging the buffer of the homogenate obtained in step iii) to a final buffer using a desalting column, and
v) passing the homogenate obtained in step iv) through a membrane filter, to obtain the pure recombinant immunogenic fusion protein in the final buffer.

In a second aspect according to the technology proposed herein, there is provided a system comprising or consisting of:
i) an anion exchange column configured to retain charged contaminants,
ii) optionally a hydrophobic interaction column configured to retain hydrophobic contaminants,
iii) a mixed mode column configured to polish the homogenate,
iv) a desalting column for buffer exchange, and
v) a membrane filter, such as a 0.2 µm filter.

Such a system is adapted to perform the method according to the first aspect of the technology proposed herein.

In the below, preferred ways of performing the technology proposed herein according to the first and second aspects are disclosed. As regards the second aspect, the parts of such a system are disclosed in connection with the first aspect of the technology proposed herein.

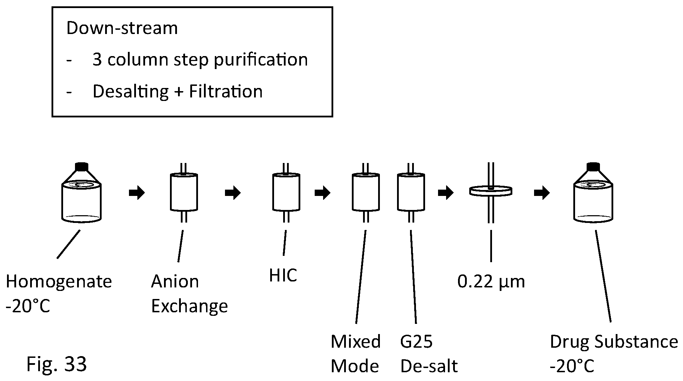

The method of the first aspect of the technology proposed herein is based on a combination of different chromatographic purification steps. An overview of the method is illustrated in Fig. 33. Chromatography allows for the separation of a mixture of molecules into separate molecules. In a chromatographic separation, a mobile phase containing the molecules to be separated from each other (dissolved in a liquid or a gas) is applied to a stationary phase (a resin) (typically placed in a column). The stationary phase may e.g. be selected to allow for separation based on size, ionic charge and/or hydrophilicity/hydrophobicity. The molecules will therefore travel through the resin at different velocities depending on their size or their binding affinity to the resin. The stationary phase may be selected to retain the molecule(s) of interest or to bind the molecule(s) that are not of interest so the molecule(s) of interest pass through the stationary phase without binding to it. By using different buffers, the properties of molecules can be affected so that the bind or do not bind the resin. If the molecules of interest bind to the resin, they can be eluted by changing the buffer in a manner that changes the properties of the bound molecules so that they no longer bind to the resin and thus can be eluted from the column.

The recombinant fusion protein according to the technology proposed herein comprises or consists of a first amino acid sequence having at least 90% sequence identity with the amino acid sequence of the N-terminal region of a first group B *Streptococcus* surface protein, said first amino acid sequence being fused to, optionally via linker, a second amino acid sequence having at least 90% sequence identity with the amino acid sequence of the N-terminal region of a second group B *Streptococcus* surface protein. The recombinant fusion protein referred to according to the technology proposed herein will be described in further detail in the below.

The recombinant fusion protein is provided in a homogenate as a starting material for the chromatographic method. The homogenate may be obtained by growing an *E. coli* comprising a gene encoding the recombinant fusion protein in a growth medium, harvesting, optionally concentrating and washing the grown cells, such as by tangential flow filtration, and disrupting the cells to prepare a homogenate comprising the recombinant fusion protein. Generally, a homogenate is a suspension of cell fragments and cell constituents obtained when tissue, in this case cells, is homogenized.

In the first step (step i)) of the method according to the first aspect of the technology proposed herein the homogenate comprising the recombinant fusion protein is passed through an anion exchange column configured to retain charged contaminants. This step is mainly designed to remove process impurities. Such an anion exchange column comprises a resin with positively charged ions. Thereby, negatively charged molecules of the homogenate bind to the resin and the positively charged molecules pass through the resin without binding. In step i), any anion exchange resin could be used. Exemplary resins may be comprised of agarose, dextran-gel or hydroxylated methacrylate polymer. The positively charged ions may be for example quaternized amine, quaternized polyethylenimine, polyethyleneimine or dimethylaminopropyl.

The anion exchange resin used in step i) may preferably be a diethylaminoethanol crosslinked beaded agarose resin, such as DEAE Sepharose which is a weak anion exchange chromatography resin. Before loading the homogenate onto the column, the homogenate may be conditioned with a buffer, such as 0.5 M Bis-Tris-HCl, pH 6.0, and filtered. The homogenate (for example in a Tris-HCl (e.g. 50 mM), pH 7.5 buffer) is loaded onto the column comprising the resin whereby the recombinant fusion protein (and other negatively charged molecules) binds to the resin while positively charged molecules pass through the column. The sample load may for example be from 10 g/L to 25 g/L, such as from 12 g/L to 20 g/L (g/L column volume). For large scale production when the sample load is higher, there is an increased risk for column fouling. This can be prevented by decreasing the flow rate. The column is thereafter washed by running a buffer through the column. Typically, 1-5, such as 3 or 4, column volumes are used for the washing step. For example, the washing step may be performed using a Tris-HCl buffer, such as 20 mM Bis-Tris-HCl, pH 6.0. The recombinant fusion protein is then eluted from the column by eluting it with an elution buffer that changes the protein's electrical charge so that it detaches from the resin. For example, this may be achieved by eluting with increasing salt concentration of the buffer used for elution. For example, the recombinant fusion protein may be eluted using a 20 mM Bis-Tris-HCl, pH 6.0 buffer, with consecutively increasing salt concentration, for example where the elution buffer in the first elution step has 60 mM NaCl, the elution buffer in the second elution step has 170 mM NaCl and the elution buffer in the third wash step has 1 M NaCl. Typically, 1-5, such as 3-4, column volumes are used for elusion with each elution buffer. Optionally, the collected eluate is filtered through a 0.2 µm filter before proceeding to the next step. The linear flow rate for the anion exchange chromatography step may e.g. be from 110 cm/h to 140 cm/h, such as from 120 cm/h to 132 cm/h. For large scale production flow rates, see examples of suitable flow rates in Table 1 below. Preferably, the residency time in the column is kept constant when scaling up the process by adjusting the flow rate.

Preferably, in particular when GBS-NN or GBS-NN2 is to be purified, the anion exchange chromatography step is performed by conditioning the homogenate with 500 mM Bis-Tris pH 6 before the sample is loaded onto a ready to process (RTP) DEAE Sepharose FF chromatography column that is pre-equilibrated in 20 mM Bis HCl, pH 6.0. The GBS-NN or GBS-NN2 protein is bound, and non-specific materials are removed using the equilibration buffer. GBS-NN or GBS-NN2 is eluted using three step gradients of 60 mM, 170 mM and 1 M NaCl in the same buffer. The product peak is eluted in 170 mM step. The product containing fractions are pooled and filtered through a 0.2 µm filter.

The parameters that determine column performance in anion exchange chromatography are well known and are maintained in a similar range as the size of the column is increased. Table 1 below shows the recommended column dimensions and operating parameters for the anion exchange column purification. As a practical measure to simplify operations at the larger scale, the specified column height is specified as a narrow range rather than a fixed value, while maintaining the same contact time as the liquid flows through the column. The sample load relative to the column size is preferably kept constant. Preferably, the residency time in the column is kept constant when scaling up the process.

**Table 1: Exemplary parameters for an upscaled anion exchange chromatography step (step i)).**

| Anion Exchange Purification Scale-up | | 20 L process | 200 L Process |
|---|---|---|---|
| Column Dimensions | | | |
| | Column diameter (cm) | 12.6 | 44* |
| | Column height (cm) | 20 | 18-22 |
| | Column volume (L) | 2.5 | 27-33 |

| Operating Parameters | | | |
|---|---|---|---|
| | Linear flow rate (cm/hr) | 120 | 108-132 |
| | Volumetric flow rate (l/hr) | 15 | 164-201 |
| | Residence time (min) | 10 | 10 |
| | Equilibration (CV) | 4 | 4 |
| | Sample load (g/)** | 12 | 12 |
| | Wash (CV) | 3 | 3 |
| | Step 1 elution (CV) | 5 | 5 |
| | Step 2 elution (CV) | 4 | 4 |
| * Standard column diameter | | | |
| ** gram protein/L column volume | | | |

The method according to the first aspect of the technology proposed herein, may optionally contain a step ii) of passing the purified homogenate obtained in step i) through a hydrophobic interaction column configured to retain hydrophobic contaminants. The hydrophobic interaction chromatography (HIC) column is mainly used as a purification step. Hydrophobic interaction chromatography (HIC) separates molecules based on their hydrophobicity and the resin used is thus a hydrophobic resin. One advantage with HIC is that allows for separation of molecules under less denaturing conditions, which is important for protein purification if the three-dimensional structure of the protein is to be maintained. In this step, if used, hydrophobic contaminants thus bind to a hydrophobic resin and are retained in the column, while the recombinant fusion protein (and other hydrophilic molecules) pass through the column.

The resin for the hydrophobic interaction chromatography has hydrophobic ligands and may e.g. be a crosslinked beaded agarose resin with a hydrophobic ligand, such as a phenyl sepharose resin, such as Phenyl Sepharose, such as Phenyl Sepharose 6 FF. The homogenate from step i) may be conditioned, such as by mixing with 100 mM NaPO₄, 2 M ammonium sulphate, pH 7.0, and filtered before loading onto the column. After loading the homogenate onto the column, the column is washed by running a buffer through the column. Typically, 1-5, such as 2-4, or 3 column volumes are used for the washing step. For example, the washing step may be performed using a NaPO₄ buffer, such as 50 mM NaPO₄, pH 7.0, 1 M ammonium sulphate. The recombinant fusion protein is then eluted from the HIC column by running an elution buffer through the column. The elution buffer may e.g. be 50 mM NaPO₄, pH 7.0. The elution is typically effected by eluting the column with 2-4, such as 3 column volumes of the elution buffer. The sample load for the HIC step may be from 15 g/L to 22 g/L, such as from 17 to 19 g/L, such as 18 g/L (g/L column volume). The eluate may be filtered through a 0.2 µm filter before proceeding to step iii). The linear flow rate for the hydrophobic interaction chromatography step may e.g. be from 110 cm/h to 140 cm/h, such as from 120 cm/h to 132 cm/h. For large scale production flow rates, see examples of suitable flow rates in Table 2 below. Preferably, the residency time in the column is kept constant when scaling up the process by adjusting the flow rate.

Preferably, in particular when GBS-NN or GBS-NN2 is to be purified the product from the AIEX step (step i)) is conditioned by adding 1+1 volume of 2 M ammonium sulphate, 100 mM sodium phosphate loading buffer, pH 7.0. The conditioned pool is loaded onto a HIC column (preferably RTP Phenyl Sepharose FF) that is pre-equilibrated in 1 M ammonium sulphate, 50 mM sodium phosphate, pH 7.0. The product is eluted using a step gradient with 50 mM sodium phosphate, pH 7.0. The product fraction is collected, and the protein-containing pool is filtered through a 0.2 µm filter.

The parameters that determine column performance during hydrophobic interaction chromatography are well known and are maintained in a similar range as the size of the column is increased. Table 2 below shows the recommended column dimensions and operating parameters for the HIC column purification. As a practical measure to simplify operations at the larger scale, the specified column height is specified as a narrow range rather than a fixed value, while maintaining the same contact time as the liquid flows through the column.

**Table 2: Exemplary parameters for an upscaled hydrophobic interaction chromatography step (step ii)).**

| HIC Purification Scale-up | | 20 L process | 200 L Process |
|---|---|---|---|
| Column Dimensions | | | |
| | Column diameter (cm) | 12.6 | 44* |
| | Column height (cm) | 20 | 18-22 |
| | Column volume (L) | 2.5 | 27-33 |

| Operating Parameters | | | |
|---|---|---|---|
| | Linear flow rate (cm/hr) | 120 | 108-132 |
| | Volumetric flow rate (L/hr) | 15 | 164-201 |
| | Residence time (min) | 10 | 10 |
| | Equilibration (CV) | 2 | 2 |
| | Sample load (g/l)** | 18 | 18 |
| | Wash (CV) | 2.5 | 2.5 |
| | Step elution (CV) | 4 | 4 |
| * Standard column diameter | | | |
| ** gram protein/L column volume | | | |

In step iii) of the method according to the technology proposed herein, the homogenate obtained in step i) or ii) is passed through a mixed mode column to polish the homogenate. "Polishing" in the context of protein purification relates to the removal of the last impurities from the homogenate. These impurities are usually proteins that have very similar properties to the protein of interest and that thus have not been removed in the preceding purification steps. Removal of these last impurities is of outmost importance if the recombinant fusion protein is to be used for therapeutic applications, such as for immunization. Initial trials using size exclusion chromatography for step iii) were unsuccessful for large scale production of the recombinant fusion protein. Thus, this step had to be modified. Mixed mode chromatography (MMC, also called multimodal chromatography) was one alternative that was tested, and this was found to give a satisfactory result. The MMC resin is configured to retain DNA, host cell proteins (HCP), leached protein A, dimers and larger aggregates, and viruses. The MMC may be performed with a MMC cation exchange resin, a MMC anion exchange resin or a MMC cation exchange and metal affinity resin. Non-limiting samples of functional groups for such resins are given in Table 3 which also gives examples of resins that are suitable to use for step iii). Size exclusion chromatography resulted in a yield of 72-82%, while with the use of MMC, the yield could be increased up to 91% (see experimental section). Replacing the size exclusion step with MMC therefore resulted in an overall increase in yield of recombinant fusion protein, see Table 14.

Preferably, a MMC anion exchange resin comprising an agarose matrix with a multimodal N-benzyl-N-methyl ethanolamine ligand, such as Capto Adhere resin is used for step iii) as this type of resin provides a high yield of recombinant fusion protein. The MMC anion exchange resin may thus comprises a resin having N-benzyl-N-methyl ethanolamine as ligand on a matrix of cross-linked agarose. Further, this type of resin also proved to be robust and to give a good product purity.

Before application onto the MMC column, the homogenate from step i) or ii) may be diluted in a buffer, such as 20 mM Bis-Tris, pH 6.0 buffer. The dilution may e.g. be from 3 times to 10 times, such as from 4 to 9 times, such as from 5 to 8 times. The MMC column may be pre-equilibrated with the same buffer as the homogenate was diluted in. The sample load for the MMC column may be from 1 g/L to 30 g/L, such as from 1 g/L to 24 g/L, such as from 1 g/L to 16 g/L (g/L column volume). As a general rule, a safe load level for the MMC can be defined as 80% of the maximum load, wherein the maximum load is defined as the load where a 10% sample breakthrough occurs. The column may be washed with 2-6, such as 3-5, such as 4 column volumes after loading the homogenate to the column. Such washing may e.g. be performed using the same buffer the homogenate was diluted in.

When using a MMC anionic exchange resin, elution of the recombinant fusion protein may be effected using a 20 mM Bis-Tris, 1 M NaCl, pH 6.0 buffer. The elution preferably is performed using a gradient from 0-100% of the buffer over a number of elution steps, such as 10-18, such as 15, column volumes, wherein the final elution step(s) are performed using 100% buffer. The gradient may for example comprise a first elution step of ca 4 column volumes of 15-20% buffer, such as 17% buffer, a second elution step of ca 8 column volumes of 50-60% buffer, such as 55% buffer, and a third elution step of ca 2-5, such as 3 or 4, column volumes of 100% buffer.

The linear flow rate in the MMC step may e.g. be from 200 cm/h to 350 cm/h, such as from 300 to 330 cm/h. For large scale production flow rates, see examples of suitable flow rates in Table 3 below. Preferably, the residency time in the column is kept constant when scaling up the process by adjusting the flow rate. The bed height for the step iii) MMC may be from 15 cm to 25 cm, such as 20 cm.

Preferably, in particular when GBS-NN or GBS-NN2 is to be purified, the product containing the pool from the HIC step is conditioned by adding 20 mM Bis-Tris pH 6 before the sample is loaded onto the mixed mode chromatography column, such as a Capto Adhere chromatography column, that is pre-equilibrated in 20 mM Bis HCl, pH 6.0. The protein is bound, and non-specific materials are removed using the equilibration buffer. GBS-NN protein is eluted using three step gradients of 170 mM, 550 mM and 1 M NaCl in the equilibration buffer. GBS-NN2 protein is eluted using three step gradients of 60 mM, 550 mM and 1 M NaCl in the equilibration buffer. The product peak (for both GBS-NN and GBS-NN2) is eluted in 170 mM step.

The parameters that determine column performance during MMC are well known and are maintained in a similar range as the size of the column is increased. Table 3 below shows the recommended column dimensions and operating parameters for the mixed mode column purification. As a practical measure to simplify operations at the larger scale, the specified column height is specified as a narrow range rather than a fixed value, while maintaining the same contact time as the liquid flows through the column. The sample load relative to the column size is kept constant.

**Table 3: Exemplary parameters for an upscaled mixed mode chromatography step (step iii)).**

| Mixed Mode Purification Scale-up | | 20 L process | 200 L Process |
|---|---|---|---|
| Column Dimensions | | | |
| | Column diameter (cm) | 12.6 | 44* |
| | Column height (cm) | 20 | 18-22 |
| | Column volume (L) | 2.5 | 27-33 |

| Operating Parameters | | | |
|---|---|---|---|
| | Linear flow rate (cm/hr) | 300 | 270-330 |
| | Volumetric flow rate (L/hr) | 37.4 | 411-502 |
| | Residence time (min) | 4 | 4 |
| | Equilibration (CV) | 3 | 3 |
| | Sample load (L)** | 24 | 24 |
| | Wash (CV) | 4 | 4 |
| | Step elution1 (CV) | 4 | 4 |
| | Step elution 2 (CV) | 8 | 8 |
| * Standard column diameter | | | |
| ** gram protein/L column volume | | | |

Step iv) of the method of the technology proposed herein comprises exchanging the buffer that the homogenate of step iii) is eluted with to the final buffer of choice, such as the buffer suitable for formulation of the recombinant fusion protein into a pharmaceutical product. Should the recombinant fusion protein already be in the final buffer after step iii) this step can of course be dispensed with. Different methods for buffer exchange was tested (diafiltration by tangential flow filtration (TFF), dialysis and desalting chromatography) as disclosed in the experimental section. TFF was surprisingly found to result in a cloudy product or a very low yield of the recombinant fusion protein. TFF was therefore rejected as a method for buffer exchange. Instead, desalting was tested for buffer exchange. Surprisingly, this gentler method turned out to result in a clear and non-cloudy product. Further, the recombinant fusion protein product could be obtained at high yields (ca 90%).

The resin used in the desalting step iv) may be a dextran resin, such as a crosslinked dextran resin, such as an epichlorohydrin crosslinked dextran resin, such as a Sephadex G25 resin. Such a resin may e.g. be provided in a HiPrep^{™} 26/10 desalting column (Cytiva). The buffer that is used for elution in step iii) is thus exchanged by binding the components of interest to the resin and then eluting them with the final buffer. Typically, the maximum load in a desalting column when used according to the method of the technology proposed herein is 25% of the column volume. The bed height may be ca 20 cm for larger scale production. The flow rate will have to be adjusted to the bed height. Using a lower flow rate may decrease sample dilution. The linear flow rate is typically from 100 cm/h to 200 cm/h, such as from 140 to 160 cm/h, such as from 143 cm/h to 157 cm/h, such as from 150 cm/h to 157 cm/h. The sample load for a desalting column may be up to 25% of the column volume or up to the maximal load volume. The sample load may e.g. be 1-20, such as 1-15, such as 1-10, such as 1-5 g/L. The desalting column may be equilibrated with the final buffer before loading the homogenate. The recombinant fusion protein is eluted from the desalting column using the final buffer. The final buffer is for example phosphate buffered saline (PBS, 7 mM Na/K-phosphate, 0.15 M NaCl, pH 7.2).

Preferably, in particular when GBS-NN or GBS-NN2 is to be purified the homogenate obtained in step iii) is loaded onto an epichlorohydrin crosslinked dextran resin, such as a G25 (Sephadex G-25 Medium) column pre-equilibrated with the final formulation buffer. The product containing fractions are pooled and filtered through a 0.2 µm filter.

The parameters that determine desalting column performance are well known and are maintained in a similar range as the size of the column is increased. Table 4 below shows the recommended column dimensions and operating parameters for a G25 de-salting column. The column length is kept within a relatively narrow defined range, as a balance between maintaining the separation efficiency of the column and providing for some variation in achieving the target column height when packing the column. Contact time as the liquid flows through the column and sample load relative to the column size are kept constant. Successful scale-up is straight forward to verify though testing of in process samples at each column step and product quality at the final bulk drug substance.

**Table 4: Exemplary parameters for an upscaled desalting chromatography step for buffer exchange (step iv)).**

| G25 de-salt Scale-up | | 20 L process | 200 L Process |
|---|---|---|---|
| Column Dimensions | | | |
| | Column diameter (cm) | 20 | 63* |
| | Column height (cm) | 20.7 | 19.7-21.7 |
| | Column volume (L) | 6.5 | 61-68 |

| Operating Parameters | | | |
|---|---|---|---|
| | Linear flow rate (cm/hr) | 150 | 143-157 |
| | Volumetric flow rate (L/hr) | 47 | 445-490 |
| | Residence time (min) | 8.3 | 8.3 |
| | Equilibration (CV) | 5 | 5 |
| | Max. sample load (L) | 1.6 | 15-17** |
| | Wash (CV) | 2 | 2 |
| | # of cycles | 4-6 | 4-6 |
| * Standard column diameter | | | |
| ** The sample load per unit column volume is kept constant | | | |

In the final step, step v), of the method of the technology proposed herein, the homogenate of step iv) is passed through a membrane filter, such as a 0.2 µm filter, to obtain the pure recombinant immunogenic fusion protein in the final buffer.

The method of the technology proposed herein may further comprise a step of adjusting the concentration and performing a final filtration. For example, the buffer exchanged product may be diluted to a target concentration of 1.75 mg/ml with formulation buffer (7 mM phosphate, pH 7.2, 150 mM NaCl) and filtered drug through a 0.2 µm filter. The filtered drug substance is preferably stored at -20 ± 5°C if storage is necessary.

For example, the method may be performed in the following manner:
Step i) Anion Exchange Chromatography (AEIX)
   The homogenate is conditioned with 500 mM Bis-Tris pH 6 before the sample is loaded onto a DEAE Sepharose chromatography column, such as a RTP DEAE Sepharose FF chromatography column that may be pre-equilibrated in 20 mM Bis HCl, pH 6.0. The recombinant fusion protein is bound, and non-specific materials are removed using the equilibration buffer. The recombinant fusion protein is eluted using three step gradients of 60 mM, 170 mM and 1 M NaCl in the 20 mM Bis HCl, pH 6.0 buffer. The product peak is eluted in 170 mM step. The product containing fractions are pooled and filtered through a 0.2 µm filter into a storage tank and kept at 2 to 8°C if necessary.
Step ii) Hydrophobic Interaction Chromatography
   The product containing pool from the AlEX step is conditioned by adding 1+1 volume of 2 M ammonium sulphate, 100 mM sodium phosphate loading buffer, pH 7.0. The conditioned pool is loaded onto a HIC column (e.g. RTP Phenyl Sepharose FF) that is pre-equilibrated in 1 M ammonium sulphate, 50 mM sodium phosphate, pH 7.0. The product is eluted using a step gradient with 50 mM sodium phosphate, pH 7.0. The product fraction is collected, and the protein-containing pool is filtered through a 0.2 µm filter and stored at 2 to 8°C if necessary.
Step iii) Mixed Mode Chromatography
   The product containing the pool from the HIC step is conditioned by adding 20 mM Bis-Tris pH 6 before the sample is loaded onto a mixed mode chromatography column, such as a Capto Adhere chromatography column, that may be pre-equilibrated in 20 mM Bis HCl, pH 6.0. The protein is bound, and non-specific materials are removed using the equilibration buffer. The GBS-NN protein is eluted using three step gradients of 170 mM, 550 mM and 1 M NaCl in the equilibration buffer. The GBS-NN2 protein is eluted using three step gradients of 60 mM, 550 mM and 1 M NaCl in the equilibration buffer. The product peak (for both GBS-NN and GBS-NN2) is eluted in 170 mM step.
Step iv) Desalting step (buffer exchange)
   The product solution from the MMC step is loaded onto a G25 (Sephadex G-25 Medium) column pre-equilibrated with the final formulation buffer. The product containing fractions are pooled and filtered through a 0.2 µm filter into a storage tank and kept at 2 to 8°C if necessary before concentration adjustment.
Step v) Final Filtration and Storage
   The buffer exchanged product is filtered through a 0.2 µm filter. The filtered drug substance bulk is aliquoted and stored at -20 + 5°C. Before or after filtration the buffer exchanged product may be diluted to a target concentration of 1.75 mg/ml with formulation buffer (7 mM phosphate, pH 7.2, 150 mM NaCl).

The method according to the first aspect of the technology proposed herein may for example comprise or consist of the steps of:
i) passing a homogenate comprising the recombinant fusion protein through an anion exchange column (optionally pre-equilibrated in 20 mM Bis HCl, pH 6.0)configured to retain charged contaminants, wherein said anion exchange column has a diethylaminoethanol crosslinked beaded agarose resin, such as DEAE Sepharose FF, and wherein said column is washed using a 20 mM Bis-Tris-HCl, pH 6.0 buffer and a 20 mM Bis-Tris-HCl, pH 6.0 buffer with gradually increasing NaCl concentration (e.g. first 60 mM, then 170 mM and finally 1 M NaCl in the buffer) is used for elution wherein the sample load is of from 10 g/L to 25 g/L, such as from 12 g/L to 20 g/L (g/L column volume) and the linear flow rate is from 110 cm/h to 140 cm/h, such as from 120 cm/h to 132 cm/h;
ii) optionally passing the homogenate of step i) through a hydrophobic interaction column (optionally pre-equilibrated in 1 M ammonium sulphate, 50 mM sodium phosphate, pH 7.0) configured to retain hydrophobic contaminants, wherein said hydrophobic interaction column has a crosslinked beaded agarose resin such as a phenyl sepharose resin, and wherein said column is washed using a 50 mM NaPO₄, pH 7.0, 1 M ammonium sulphate buffer and a 50 mM NaPO₄, pH 7.0 buffer is used for elution wherein the sample load is from 15 g/L to 22 g/L, such as from 17 to 19 g/L, such as 18 g/L (g/L column volume) and the linear flow rate is from 110 cm/h to 140 cm/h, such as from 120 cm/h to 132 cm/h and wherein the pooled eluate is filtered through a 0.2 µm filter;
iii) passing the homogenate of step i) or ii) through a mixed mode column to polish the homogenate, wherein said mixed mode column has an anion exchange resin (optionally pre-equilibrated in 20 mM Bis HCl, pH 6.0), such as Capto Adhere, the column washed with 20 mM Bis HCl, pH 6.0, and a gradient of from 0-100% of a 20 mM Bis-Tris, 1 M NaCl, pH 6.0 buffer is used for elution (for GBS-NN three step gradients of 170 mM, 550 mM and 1 M NaCl in 20 mM Bis HCl, pH 6.0 and for GBS-NN2 three step gradients of 60 mM, 550 mM and 1 M NaCl in 20 mM Bis HCl, pH 6.0 ) wherein the sample load is from 1 g/L to 30 g/L, such as from 1 g/L to 24 g/L, such as from 1 g/L to 16 g/L (g/L column volume) and the linear flow rate is from about 200 cm/h to about 350 cm/h, such as from about 300 to about 330 cm/h;
iv) exchanging the buffer of the homogenate of step iii) to the final buffer using a desalting column, wherein said desalting column has a dextran resin, such as a crosslinked dextran resin, such as an epichlorohydrin crosslinked dextran resin, such as a Sephadex G25 resin and wherein the final buffer in the form of 7 mM Na/K-phosphate, 0.15 M NaCl, pH 7.2 is used for elution wherein the sample load may e.g. be 1-20, such as 1-15, such as 1-10, such as 1-5 g/L and the linear flow rate is 100-200 cm/h, such as from about 140 to about 160 cm/h, such as from about 143 cm/h to about 157 cm/h, such as from about 150 cm/h to about 157 cm/h;
v) passing the homogenate of step iv) through a 0.2 µm membrane filter, to obtain the pure recombinant immunogenic fusion protein in the final buffer.

As the skilled person is aware of, the flow rate in the different method steps will have to be adjusted to the bed height and a higher flow rate can be used for higher beds.

For the anion exchange, hydrophobic interaction and mixed mode chromatography steps, typically, based on the chromatogram at UV280 nm, the pools with the most target protein are pooled for further purification. A suitable cut off level is about 10% of the peak top UV value wherein cut-off fractions are included in the pool.

In the desalting step, the pooling strategy typically is to collect peak based on UV280, pH and conductivity chromatogram by starting collection when UV starts to rise and ending collection when pH start to decrease and before conductivity peak starts to rise above baseline or UV280 comes close to baseline.

Exemplary ways of performing the different method steps of the method of the technology proposed herein are given in the Experimental section. The skilled person is aware of that some modifications of these method steps can be performed while still getting a similar resulting product. However, the Experimental section provides preferred ways of performing the different method steps.

The recombinant fusion protein to be produced by the method of the technology proposed herein comprises a first amino acid sequence having at least 90% sequence identity with the amino acid sequence of the N-terminal region of a first group B *Streptococcus* surface protein. The first amino acid sequence is fused to, optionally via linker, a second amino acid sequence having at least 90% sequence identity with the amino acid sequence of the N-terminal region of a second group B *Streptococcus* surface protein. The recombinant fusion protein is preferably an immunogenic fusion protein, i.e. a fusion protein able to elicit a protective immunity against group B *Streptococcus,* such as by vaccination. The first and second group B *Streptococcus* surface protein are preferably not the same. The recombinant fusion protein preferably consists of said first and second group B *Streptococcus* surface proteins and optionally a linker.

The first and second group B *Streptococcus* surface protein may be selected from the group consisting of Rib protein, Alp1 protein, Alp2 protein, Alp3 protein, Alp4 protein and AlpC protein, with the proviso that the first and second group B *Streptococcus* surface proteins are preferably not the same. Alternatively, the recombinant fusion protein comprises at least one amino acid sequence having at least 90% sequence identity with the amino acid sequence of the N-terminal region of the group B *Streptococcus* surface protein Rib, Alp1, Alp2, Alp3, Alp4 and AlpC.

For example, the recombinant fusion protein may be a fusion protein comprising the N-terminal regions of the Group B *Streptococcus* Rib and AlpC proteins or the N-terminal regions of the Group B *Streptococcus* Alp1 and Alp2 proteins.

Preferably, the recombinant fusion protein consists of: a first amino acid sequence part consisting of 170 to 178 amino acids, preferably 174 to 175 amino acids, and having at least 90% sequence identity with the amino acid sequence shown in SEQ ID NO: 1 and a second amino acid sequence part consisting of 165 to 174 amino acids, preferably 169 to 170 amino acids, and having at least 90% sequence identity with the amino acid sequence shown in SEQ ID NO: 2 and optionally a linker amino acid sequence part consisting of 1 to 20 amino acids and separating the first amino acid sequence part from the second amino acid part, and wherein the fusion protein preferably consists of 335 to 372 amino acids, preferably 343 to 353 amino acids, more preferably 343 to 347 amino acids. Further, details of such a recombinant fusion protein can be found in WO2022/207657.

The recombinant fusion protein may preferably have an amino acid sequence of SEQ ID NO: 3, or an amino acid sequence having at least 90%, at least 92%, at least 94%, at least 96%, at least 98%, at least 99%, or 100% identity thereto. This recombinant fusion is a fusion protein between the N-terminal regions of Rib and AlpC with an EF linker, herein also denoted GBS-NN or NN-GBS.

Alternatively, the recombinant fusion protein consists of: a first amino acid sequence part consisting of 169 to 177 amino acids, preferably 172 to 173 amino acids, and having at least 90% sequence identity with the amino acid sequence shown in SEQ ID NO: 4 and a second amino acid sequence part consisting of 168 to 178 amino acids, preferably 171 to 172 amino acids, and having at least 90% sequence identity with the amino acid sequence shown in SEQ ID NO: 5 and optionally a linker amino acid sequence part consisting of 1 to 20 amino acids and separating the first amino acid sequence part from the second amino acid part, and wherein the fusion protein preferably consists of 338 to 375 amino acids, preferably 343 to 353 amino acids, more preferably 343 to 347 amino acids. Further, details of such a recombinant fusion protein can be found in WO2022/207657.

The recombinant fusion protein may preferably have an amino acid sequence of SEQ ID NO: 6 or an amino acid sequence having at least 90%, at least 92%, at least 94%, at least 96%, at least 98%, at least 99%, or 100% identity thereto. This recombinant fusion protein is a fusion protein between the N-terminal regions of Alp1 and Alp2 with an EF linker, herein also denoted GBS-NN2 or NN2-GBS.

The first and second amino acid sequences may preferably be separated by a linker. This linker is typically 1-20 amino acids long, preferably 1 to 10 amino acids, preferably 2 to 5 amino acids, more preferably 2 amino acids, and most preferably the linker consist of the amino acid sequence EF. The function of the linker is to join the first and second group B *Streptococcus* N-terminal regions. The linker is typically a linker that is not cleavable.

Whenever an amino or nucleic acid sequence is referred to herein, this also includes a variant of said amino or nucleic acid sequence, wherein said variant has a sequence identity of at least 95%, such as at least 96%, such as at least 97%, such as at least 98%, such as at least 99% or 100% identity to the sequence referred to. According to the technology proposed herein, sequence identity is calculated in the following manner. By an amino or nucleic acid sequence having a sequence with at least, for example 95%, identity to a reference sequence, is intended that the sequence is identical to the reference sequence except that the sequence may include up to 5 amino or nucleic acid differences per each 100 amino or nucleic acids of the reference sequence. In other words, to obtain an amino or nucleic acid sequence having a sequence identity of at least 95%, up to 5% of the amino or nucleic acids in the reference sequence may be deleted or substituted with another amino or nucleic acid, or a number of amino or nucleic acids up to 5% of the total number of amino or nucleic acids in the reference sequence may be inserted into the reference sequence. If the two sequences to be compared are not of equal length, they must be aligned to best possible fit, and the sequence identity is calculated over the length of the reference sequence. These amino or nucleic acid changes of the reference sequence may occur at the amino and/or carboxy terminal positions for an amino acid sequence or at the 5' and/or 3' end for a nucleic acid sequence of a reference amino or nucleic acid sequence or anywhere between those terminal positions, interspersed either individually within the reference sequence or in one or more contiguous groups within the reference sequence.

### SEQUENCES

(methionine in the N-terminal end is always optional and not to be included when sequence identities are calculated):
SEQ ID NO: 1 (N-terminal region of Rib) 
MAEVISGSAVTLNTNMTKNVQNGRAYIDLYDVKNGKIDPLQLITLNSPDLKAQYVIRQGG
NYFTQPSELTTVGAASINYTVLKTDGSPHTKPDGQVDIINVSLTIYNSSALRDKIDEVKKKA
EDPKWDEGSRDKVLISLDDIKTDIDNNPKTQSDIANKITEVTNLEKILVPRIP
SEQ ID NO: 2 (N-terminal region of AlpC) 
STIPGSAATLNTSITKNIQNGNAYIDLYDVKLGKIDPLQLIVLEQGFTAKYVFRQGTKYYGD
VSQLQSTGRASLTYNIFGEDGLPHVKTDGQIDIVSVALTIYDSTTLRDKIEEVRTNANDPK
WTEESRTEVLTGLDTIKTDIDNNPKTQTDIDSKIVEVNELEKLLVLS
SEQ ID NO: 3 (GBS-NN): 
MAEVISGSAVTLNTNMTKNVQNGRAYIDLYDVKNGKIDPLQLITLNSPDLKAQYVIRQGG
NYFTQPSELTTVGAASINYTVLKTDGSPHTKPDGQVDIINVSLTIYNSSALRDKIDEVKKKA
EDPKWDEGSRDKVLISLDDIKTDIDNNPKTQSDIANKITEVTNLEKILVPRIPEFSTIPGSAA
TLNTSITKNIQNGNAYIDLYDVKLGKIDPLQLIVLEQGFTAKYVFRQGTKYYGDVSQLQST
 
GRASLTYNIFGEDGLPHVKTDGQIDIVSVALTIYDSTTLRDKIEEVRTNANDPKWTEESRT
EVLTGLDTIKTDIDNNPKTQTDIDSKIVEVNELEKLLVLS
SEQ ID NO: 4 (N-terminal region of Alp1) 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLP
SEQ ID NO: 5 (N-terminal region of Alp2) 
STIPGSAATLNTSITKNIQNGNAYIDLYDVKNGLIDPQNLIVLNPSSYSANYYIKQGAKYYS
NPSEITTTGSATITFNILDETGNPHKKADGQIDIVSVNLTIYDSTALRNRIDEVINNANDPKW
SDGSRDEVLTGLEKIKKDIDNNPKTQIDIDNKINEVNEIEKLLVVSL
SEQ ID NO: 6 (GBS-NN2, N-terminal Met removed post-translationally): 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLPEFSTIPGSAATLN
TSITKNIQNGNAYIDLYDVKNGLIDPQNLIVLNPSSYSANYYIKQGAKYYSNPSEITTTGSA
TITFNILDETGNPHKKADGQIDIVSVNLTIYDSTALRNRIDEVINNANDPKWSDGSRDEVLT
GLEKIKKDIDNNPKTQIDIDNKINEVNEIEKLLVVSL
SEQ ID NO: 7 (codon optimized GBS-NN gene): 
ATGGCCGAAG TTATTAGCGG TAGCGCAGTT ACCCTGAATA CCAATATGAC
CAAAAATGTG CAGAATGGCC GTGCCTATAT TGATCTGTAT GATGTGAAAA
ATGGCAAAAT CGATCCGCTG CAGCTGATTA CCCTGAATTC TCCGGATCTG
AAAGCACAGT ATGTTATTCG TCAGGGTGGT AACTATTTTA CCCAGCCGAG
CGAACTGACC ACCGTTGGTG CAGCAAGCAT TAACTATACC GTTCTGAAAA
CCGATGGTAG TCCGCATACC AAACCGGATG GTCAGGTTGA TATTATCAAT
GTTAGCCTGA CCATCTATAA TAGCAGCGCA CTGCGTGATA AAATTGATGA
AGTGAAAAAG AAAGCCGAAG ATCCGAAATG GGATGAAGGT AGCCGTGATA
AAGTTCTGAT TAGCCTGGAT GATATCAAAA CCGATATTGA TAACAACCCG
AAAACCCAGA GCGATATTGC AAACAAAATT ACCGAAGTGA CCAATCTGGA
AAAAATTCTG GTTCCGCGTA TTCCGGAATT TAGCACCATT CCGGGTAGCG
CAGCAACACT GAATACAAGC ATTACCAAAA ACATTCAGAA CGGCAACGCA
TATATCGATC TGTACGACGT TAAACTGGGT AAAATTGATC CTCTGCAACT
 
GATCGTTCTG GAACAGGGTT TTACCGCAAA ATATGTGTTT CGTCAGGGCA
CCAAATACTA TGGTGATGTT AGCCAGCTGC AGAGCACCGG TCGTGCAAGC
CTGACCTATA ACATTTTTGG TGAAGATGGT CTGCCGCATG TTAAAACCGA
TGGCCAGATT GATATCGTTA GCGTTGCACT GACGATTTAT GATAGCACCA
CCCTGCGCGA TAAAATCGAA GAAGTTCGTA CCAACGCAAA CGATCCTAAA
TGGACCGAAG AAAGCCGTAC CGAAGTTCTG ACCGGTCTGG ATACCATTAA
AACGGATATC GACAATAATC CTAAAACGCA GACAGATATT GATAGCAAAA
TTGTGGAAGT GAATGAACTGGAAAAACTGC TGGTTCTGAG CTAATAA
SEQ ID NO: 8 (codon optimized GBS-NN2 gene): 
ATGGCCGAAGTTATTAGCGGTAGCGCAGCAACCCTGAATAGCGCACTGGTTAAAAAT
GTTAGCGGTGGCAAAGCCTATATCGACATCTATGATGTGAAAAACGGCAAAATTGAT
CCGCTGAATCTGATTGTTCTGACCCCGAGCAATTATAGCGCCAACTATTATATCAAAC
AGGGTGGTCGTATTTTCACCAGCGTTAATCAGCTGCAGACACCGGGTACAGCAACCA
TTACCTATAACATTCTGGATGAAAATGGCAACCCGTATACCAAAAGTGATGGCCAGAT
TGATATTGTTAGCCTGGTTACCACCGTTTATGATACCACCGAACTGCGCAATAACATC
AACAAAGTTATTGAGAATGCCAACGACCCGAAATGGTCAGATGATAGCCGTAAAGAT
GTTCTGAGCAAAATCGAGGTGATCAAAAACGATATTGATAACAACCCGAAAACCCAG
AGCGATATCGACAACAAAATTGTGGAAGTGAACGAGCTGGAAAAACTGCTGGTTCTG
CCGGAATTTAGCACCATTCCGGGTTCAGCAGCCACACTGAATACCAGCATTACCAAA
AACATTCAGAATGGCAACGCCTACATTGATCTGTACGATGTAAAAAATGGTCTGATCG
ATCCGCAGAACCTGATCGTGCTGAATCCGAGCAGCTATTCAGCCAATTATTATATTAA
ACAAGGCGCAAAATACTATAGCAACCCGAGCGAAATTACCACCACCGGTAGCGCCA
CCATTACGTTTAATATCCTGGACGAAACCGGTAACCCGCATAAAAAAGCAGATGGTC
AAATTGATATCGTGAGCGTTAACCTGACGATTTATGATAGCACAGCCCTGCGTAATC
GTATTGATGAAGTGATTAATAACGCGAATGATCCTAAATGGTCCGATGGTAGTCGTG
ATGAAGTACTGACCGGTCTGGAAAAAATCAAAAAAGACATCGACAATAATCCGAAAA
CGCAGATTGACATTGACAATAAAATCAACGAGGTGAACGAGATCGAGAAACTGCTGG
TAGTTAGCCTGTAATAA
SEQ ID NO: 9 (Alp4N):

VVEGSAATLNTAMTKNMQNGNAYIDIYDVKLGKIDPLQLIKLEPGYTAIYYITQGSKVYA
NVSELQTPGAAKVNYRIQTSDGSDHIKSDGQLDSVNISLTVYD

The technology proposed herein will be further described in the following examples, which do not limit the scope of the invention described in the claims.

### EXPERIMENTAL SECTION

The theoretical isoelectric point (pl) of NN_GBS is ca 4.8 and it was decided to examine a combination of anion exchange chromatography and hydrophobic interaction chromatography (HIC). First, the pl was determined using isoelectric focusing of both a purified, tagged NN_GBS and the extract of the non-tagged, non-purified NN_GBS. Based on analysis isoelectrical focusing on Isogel Agarose IEF (Lonsa) and the resulting plot of pl vs. migration a pl of 4.9 was determined, in good agreement with the theoretical estimate.

The following experiments were then performed, as further detailed below.

Example 1 is directed to optimization of anion exchange chromatography (step i) as regards the choice of column (resin) to use.

Example 2 is directed to optimisation of hydrophobic interaction chromatography (step ii) as regards the choice of column (resin) to use.

Example 3 is directed to an upscaled chromatographic method for purifying a homogenate comprising a recombinant fusion protein as regards steps i) and ii) (anion exchange chromatography and hydrophobic interaction chromatography, respectively).

Example 4 is directed to the development of purification process suitable for large scale production of recombinant fusion proteins GBS-NN and GBS-NN2, including steps i) and ii) and the development of a step replacing a size exclusion chromatography, which resulted in steps iii) and iv) of the method of the technology proposed herein.

Example 5 is directed to further optimizations of the anion mixed mode chromatography (step iii, polishing step) as regards determination of suitable start material dilution, development of step elution parameters, flow rate and resin capacity testing.

Example 6 is directed to the optimization of column bed height, elution step and flow rate for the anion mixed mode chromatography step (step iii, polishing step).

Example 7 is directed to further developments of the polishing step (step iii) as regards load range.

Example 8 is directed to the development of formulation step (step iv), i.e. to provide the recombinant fusion protein in a buffer suitable for formulation as a pharmaceutical product.

The experiments were performed with the recombinant fusion proteins GBS-NN and/or GBS-NN2. The start material for the purification is GBS-NN or GNS-NN2 homogenate prepared by fermentation of *E. coli* expressing the GBS-NN recombinant fusion protein, harvesting, concentrating and washing the cells by tangential flow filtration and disrupting the cells.

### EXAMPLE 1: Optimization of anion exchange chromatography: comparison of different anion exchange columns (resins)

According to recommendations from the supplier, GE Healthcare, the pH value selected for anion exchange chromatography should be at least 1 pH unit above the pl, in order to ensure binding to the resin. Therefore, the following two buffers were chosen:
Buffer A: 20mM BisTris-HCl pH 6
Buffer B: 20 mM BisTris-HCl pH 6 + 1 M NaCl

The following 5 ml prepacked HiTrap columns from the supplier were tested:
- DEAE Sepharose FF
- Q Sepharose FF
- Capto Q Sepharose
- Q XL Sepharose

The conditions used for chromatography were:
System: Äkta purifier 10 (GE Healthcare)
Temperature: Room temperature (approx. 22°C).
Load: 2 ml of extract, Bioneer # ACS11022
Flow rate: 5 ml/min (loading at 1 ml/min).
Wash: 5 column volumes (CV) = 25 ml.
Salt gradient for elution: 0-100% buffer B over 15 CV = 75 ml
Washing: 5 CV buffer A
Gradient: 0-100 % B, 15 column volumes

Due to a slightly high back pressure, most chromatography experiments were finalised at 3 or 4 ml/min.

Tests were performed with the recombinant fusion protein NN_GBS.

It could be concluded that with all resins, elution of NN_GBS is observed at about 20% buffer B. However, as judged by SDS-PAGE, a higher content of impurities was observed for Capto Q and Q XL Sepharose. Also, in terms of separation between product elution and the large peak (of unknown origin) observed at the beginning of each gradient, DEAE Sepharose and Q Sepharose are superior. On the basis of these experiments, DEAE Sepharose was chosen as the resin for anion exchange chromatography for further studies. DEAE Sepharose is a diethylaminoethanol crosslinked beaded agarose resin.

### Modified and up-scaled DEAE Sepharose chromatography

Since the elution was observed at relatively low concentrations of NaCl, the gradient was modified slightly and the experiment performed again on a 20 ml column.

### Conditions:

Column: 20 ml DEAE Sepharose HiPrep
System: Äkta purifier 10 (GE Healthcare)
Temperature: Room temperature (approx. 22°C)
Load: 2 ml of extract, Bioneer # ACS11022
Flow rate: 5 ml/min (loading at 1 ml/min)
Wash: 5 column volumes (CV) = 25 ml
Salt gradient for elution: 0-50% buffer B over 15 CV = 75 ml.

In this way, separation of product and an impurity previously visible as a shoulder was improved. This modified method was therefore used for future anion exchange steps. In order to produce material for hydrophobic interaction chromatography, an up-scaled experiment was carried out:
Column: 20 ml DEAE Sepharose HiPrep
System: Äkta purifier 10 (GE Healthcare)
Temperature: Room temperature (approx. 22°C)
Load: 24 ml of extract, Bioneer # ACS11022
Flow rate: 5 ml/min (loading at 1 ml/min)
Wash: 5 column volumes (CV) = 25 ml
Salt gradient for elution: 0-50% buffer B over 15 CV = 75 ml

Using this modified protocol, elution is still observed at 0.2 M NaCl. SDS-PAGE was used to confirm the presence of product and which fractions to pool.

### EXAMPLE 2: Optimization of hydrophobic interaction chromatography: comparison of different hydrophobic interaction chromatography columns (resins)

For hydrophobic interaction chromatography (HIC), the following pre-packed 1 ml columns from the HiTrap HIC selection kit (Prod. # 28-4110-07) from GE Healthcare were examined:
- Octyl FF
- Phenyl FF high sub
- Phenyl FF low sub

Buffers chosen for chromatography were:
Buffer A: 50 mM sodium phosphate pH 7 + 1 M ammonium sulphate
Buffer B: 50 mM sodium phosphate pH 7

The conditions used were:
System: Äkta purifier 10 (GE Healthcare)
Temperature: Room temperature (approx. 22°C)
Load: 0.5 ml step one (fractions C6 +C7) product mixed with 4.5 ml Buffer A
Flow rate: 1 ml/min
Wash: 5 column volumes (CV) = 5 ml
Salt gradient for elution: 0-100% buffer B over 15 V = 15 ml

On the Octyl resin, only modest retention is observed since product elution starts immediately as the ionic conductivity decreases. On Phenyl low sub, the peak maximum is seen at about 50% Buffer B while Phenyl high sub as expected elicited stronger protein binding and the peak maximum was not observed until the concentration of Buffer B had reached 100%. Therefore, Phenyl low sub was chosen as HIC matrix. Phenyl low sub is a Phenyl Sepharose resin.

In order to allow for size exclusion analysis, which can be disturbed by the presence of a salting-out agent like ammonium sulphate, a step gradient was also used, allowing elution at 100% buffer B.

### Analysis using SDS-PAGE and size exclusion chromatography

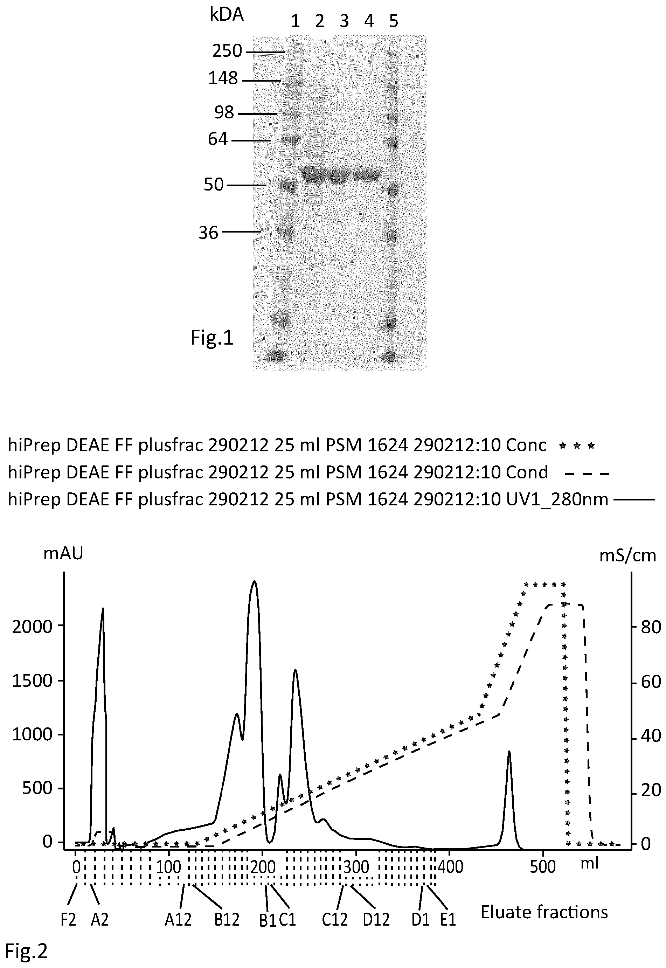

The cell extract, anion exchange product and the HIC product were analysed using SDS-PAGE and size exclusion chromatography. SDS-PAGE showed the efficient purification of the anion exchange step, resulting in a highly pure protein preparation (Fig. 1. With the amounts of protein loaded here, differences in purity between anion exchange product and the HIC product are not visible (Fig. 1).

Size exclusion chromatography showed a large peak at 1.31, which according to a calibration plot using globular proteins suggests a M_{W} of about 60 kDa. This discrepancy indicates that NN_GBS is a rather elongated molecule, in agreement with its two-domain architecture. The chromatogram of the anion exchange product is already highly pure, while the chromatogram of the HIC product prepared using the gradient method showed the presence of apparent aggregates eluting in the void volume at about 0.9 ml. This is likely due to a salting out effect on the column. This was further verified in later experiments. The size exclusion chromatogram of the HIC product prepared using the step method only showed the NN_GBS peak (Data not shown.).

### EXAMPLE 3: Upscaling of purification process as regards steps i) and ii)

An overview of the up-scaled purification process is shown below:
1.Bradford assay of crude extract to be used for the purification
2.Step one purification on DEAE column
3.Analytics after step one:
   - SDS-PAGE of loaded extract and collected fractions, Coomassie and silver staining
   - SDS-PAGE, to estimate NN-GBS recovery and purity
   - Bradford protein assay
   - Analytical size exclusion chromatography (SEC)
   - Endotoxin assay
4.Step two purification on Phenyl Sepharose column
5.Analytics after step two:
   - SDS-PAGE of step one pool loaded on the column and collected fractions, Coomassie and silver staining
   - SDS-PAGE, to estimate NN-GBS recovery
   - Bradford protein assay
6.Buffer exchange of the pool after step two to phosphate buffered saline (PBS) and Tris buffered saline (TBS)
7.Analytics of the final product, buffer exchanged to PBS or TBS:
   - SDS-PAGE to show purity, PBS product
   - Bradford protein assay, PBS and TBS products
   - Analytical SEC, PBS product
   - Endotoxin assay, PBS and TBS products
8.Dilution and distribution in vials, storage in freezer

Bradford protein assay of the crude *E. coli* extract to be used for the purification of NN_GBS showed a concentration of 20.48 mg/ml. The total protein content in the total volume of extract obtained, 730 ml, was therefore 20.48 mg/ml x 730 ml = 14.95 gram.

The Bradford protein assay data were used for calculation of the amount of extract to be used for the up-scaled purification: The desired yield of final product was 100- 200 mg, the purity of NN_GBS in crude extract was approx. 50%, the total expected recovery in steps 1 and 2 was approx. 60%. Based on these figures, an extract volume of 17-33 ml should be used for the purification.

### Step one: Anion exchange chromatography on a 20-ml DEAE Sepharose FF column (HiPrep DEAE)

The chromatographic conditions used were identical to those used above in Example 1, except that the flow rate was reduced from 5 ml/min to 2 ml/min:
System: Äkta purifier 100 (GE Healthcare).
Column: HiPrep DEAE FF 16/10, 1.6 x 10 cm, 20 ml column volume, GE Healthcare cat. no. 28-9365-41.
Buffer A: 20 mM BisTris-HCl, pH 6.0.
Buffer B: Buffer A with 1 M NaCl.
Temperature: Room temperature (approx. 22°C).
Load: 25 ml of extract, Bioneer # ACS11022, corresponding to 25 ml x 20.48 mg/ml = 512 mg.
Sample preparation before load: The extract was centrifuged in a Sorvall SS-34 rotor at 10,000 x g for 20 min. The supernatant was sterile filtered through a 0.45-µm filter and loaded.
Flow rate: 2 ml/min (60 cm/h).
Wash: 5 column volumes (CV) = 100 ml.
Salt gradient for elution: 0-50% buffer B over 15 CV = 300 ml
Fractions collected: 10 ml (load and wash), 7 ml (gradient elution).

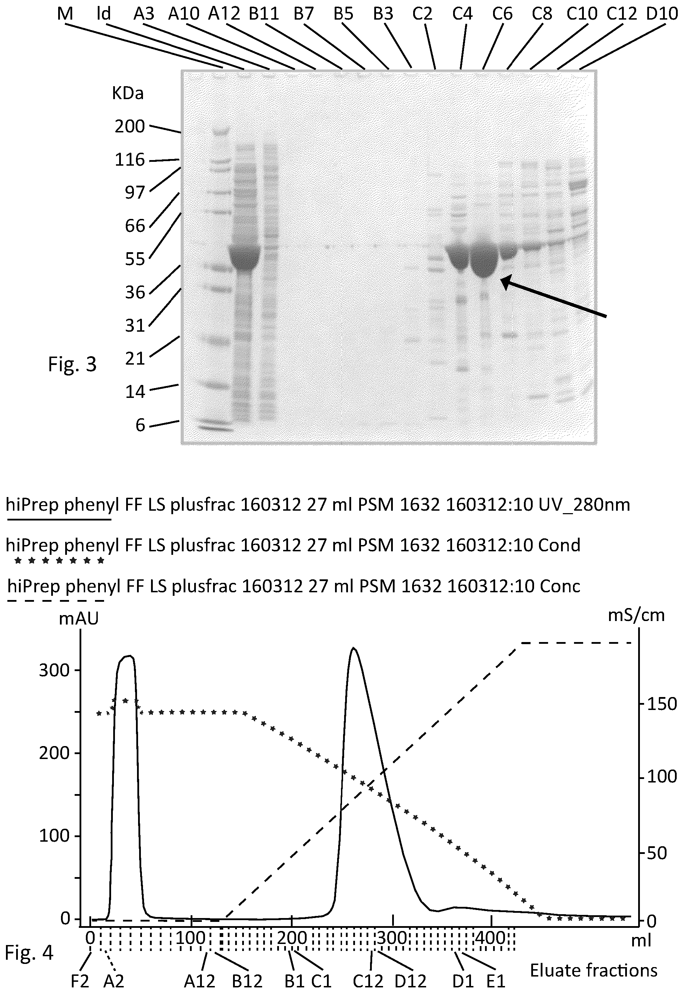

The chromatogram is shown in Fig. 2. The loaded extract and selected fractions were analysed by SDS-PAGE, see Fig. 3. Samples were reduced and run on Novex NuPAGE 12% homogeneous Bis-Tris gels (Life Technologies cat. no. NP0342BOX) using MOPS SDS running buffer (Life Technologies cat. no. NP0001). The gels were run according to the instructions of the manufacturer. The gel was stained with Coomassie blue using the Colloidal Blue staining kit (Life Technologies cat. no. LC6025).

The fraction with most NN_GBS protein were pooled.

The pool was analyzed by
1. SDS-PAGE two-fold dilutions, to analyze the NN_GBS purity
2. Total protein determination by Bradford protein assay
3. Analytical size exclusion chromatography (SEC). To show the effect of ammonium sulphate on the SEC UV-profile, the pool was analyzed both without and with addition of 1 M ammonium sulphate prior to injection on the column.
4. Endotoxin assay

SDS-PAGE of two-fold serial dilutions of loaded *E. coli* extract and pooled fractions, Coomasssie blue staining of the gel and scanning of the gel using a Gel Doc EZ Imager (Bio-Rad) showed approx. 42% NN_GBS purity in the crude extract and approx. 82 % purity in the pool after step one, data not shown. These scanning results are based on the assumption that all proteins in the sample will bind the staining reagent with equal efficiencies.

Bradford protein assay of the step one pool (volume 27.66 ml) showed a protein concentration of 8.22 mg/ml, 27.66 ml, 227 mg. Data from SDS-PAGE and Bradford assay are summarized in Table 5.

Analytical SEC of the pool after step one was run on a Superose 12 PC 3.2/30 column (GE Healthcare cat. no. 17-0674-01) with dimensions 3.2 mm x 300 mm, corresponding to a column volume of 2.4 ml. The eluent was phosphate buffered saline (PBS) prepared by Bioneer. The analysis was run on a SMART micro-chromatography system (GE Healthcare) with UV monitoring at 214 nm, 280 nm and 300 nm. Loop size was 50 µl. Injected volume was 50 µl. Two injections showing identical profiles were run. The retention volume of the NN_GBS peak (1.30 ml) corresponded to a native molecular weight of approx. 61 kDa (calibration plot not shown). The molecular weight observed by analytical SEC was significantly higher than predicted from the amino acid sequence and observed in SDS-PAGE: 38.4 kDa. The reason for this difference is not known, but could be due to NN_GBS being a rather elongated molecule. To illustrate the effect of the presence of ammonium sulphate, analytical SEC was also done with addition of 1 M ammonium sulphate to the sample prior to injection. Evidently, the presence of 1 M ammonium sulphate dramatically changed the elution profile. The test showed that samples with ammonium sulphate, e.g. from hydrophobic interaction chromatography, should be desalted prior to SEC analysis. (Data not shown.)

The endotoxin concentration in the *E*. *coli* extract loaded in step one and in the step one pool (anion exchange) was analyzed using the PyroGene recombinant Factor C assay (Lonza). The assay was done according to the instructions of the manufacturer. Samples were diluted with neutral, dilute buffer to obtain an assay response within the range of the standard curve. The endotoxin level was reduced from approx. 300,000 EU/mg to 3 EU/mg, corresponding to a 100,000-fold reduction (5 logs). This result indicated that, anion exchange chromatography, especially on a DEAE column, is very efficient for reduction of endotoxin content.

**Table 5: SDS-PAGE and Bradford assay analyses. Two step purification of NN-GBS from crude E.coli extract.**

| | Total protein Conc. (1) mg/ml | Volume ml | Amount of protein (2) mg | Yield accum. (3) % | Purity SDS-PAGE % | Purity analytical SEC (4) % | Accum. Recover y of NN-GBS (5) % |
|---|---|---|---|---|---|---|---|
| Crude extract loaded step one | 20.48 | 25.00 | 512 | 100 | 42 | - | 100 |
| Pool after step one | 8.22 | 27.66 | 227 | 44 | 82 | - | 87 |

| | | | | | | | |
|---|---|---|---|---|---|---|---|
| (1) Determined by Bradford protein assay using bovine gamma globulin as a standard (Bio-Rad). All samples were analyzed the same day using the same standard curve. (2) Calculation from Bradford assay result and volume. (3). Based on the protein amounts measured by Bradford assay. (4) the same purity was observed for the 214-nm and the 280-nm profile. (5) based on Bradford protein assay and purity estimated by SDS-PAGE. | | | | | | | |

Summary and conclusions after step one: DEAE Sepharose FF column development The chromatographic profile shown above is similar to the profile obtained earlier during initial up-scaling.

As observed earlier, the NN_GBS product eluted at ~20% buffer B, corresponding to ~200 mM NaCl.

The purity of SDS-PAGE after step one was 82% as determined by gel scanning. Recovery of NN_GBS, based on Bradford protein assay and purity estimated by SDS-PAGE, was approximately 87%.

Endotoxin content was efficiently reduced approx. 5 logs to 3 EU/mg.

In analytical size exclusion chromatography NN_GBS eluted at a position corresponding to 61 kDa, which is different from the migration observed in SDS-PAGE and the molecular weight deduced from the amino acid sequence of NN_GBS (38.4 kDa). The SEC clearly showed that samples containing ammonium sulphate should be desalted before analysis.

### Step two: Hydrophobic interaction chromatography on 20-ml Phenyl Sepharose FF low sub column

System: Äkta purifier 100 (GE Healthcare). To maintain the low level of endotoxin found in the product of step one, the Äkta purifier chromatography system was sanitized before being used for the step two in the purification: All relevant buffer inlets and outlets (A11, A12, A2, B1 and B2) were washed with 1 M NaOH and the system was then left over night. The following day, the system was washed with Milli-Q water. The sample pump and the column were also cleaned with 1 M NaOH, left for 2 hours and then washed with water.

Compared to the test of 1-ml HIC columns described above, the column volume was increased to 20 ml. All other chromatographic conditions were maintained (buffers and gradient, type of column packing material).

The following chromatographic conditions were used:
Column: HiPrep Phenyl FF (low sub) 16/10, 1.6 x 10 cm, 20 ml column volume, GE Healthcare cat. no. 28-9365-46.
Buffer A: 50 mM Na-phosphate, 1 M ammonium sulphate, pH 7.0.
Buffer B: 50 mM Na-phosphate., pH 7.0.
Temperature: Room temperature (approx. 22°C).
Load: Pool from step one. A sample was taken out for analytical SEC. The remaining volume, 26.48 ml, was used for step-two purification.
Sample preparation before load: Ammonium sulphate was added to the step one pool to obtain a final concentration of 1 M. The mixture was centrifuged at 1,500 rpm (390 x g) for 5 min, and the supernatant was loaded on the column.
Flow rate: 2 ml/min (60 cm/h)
Wash: 5 column volumes (CV) = 100 ml
Salt gradient for elution: 0-100% buffer B over 15 CV = 300 ml
Fractions collected: 10 ml (load and wash), 7 ml (gradient elution).

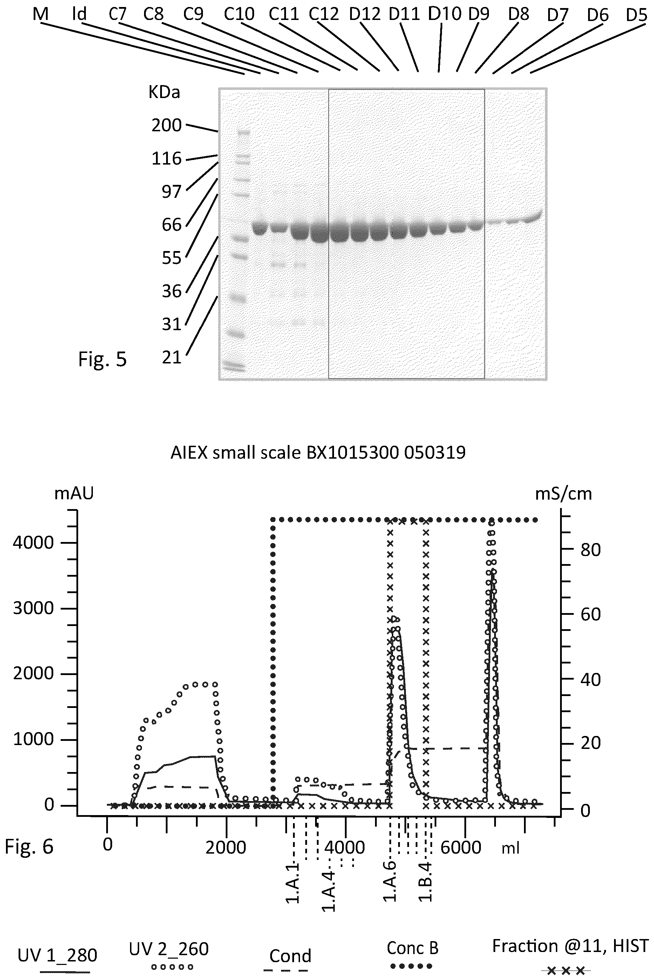

The chromatogram is shown in Fig. 4. The loaded extract and selected fractions were analyzed by SDS-PAGE as above and Coomassie blue staining of the gel (Fig.5). It was decided to pool fractions C10-C11-C12-D12-D11-D10-D9-D8, see Fig. 5. The pool, labelled PSM1640, was analyzed by total protein determination by Bradford protein assay. The yield of NN_GBS was 2.34 mg/ml, 57.62 ml, total amount 135 mg.

### Buffer exchange by gel filtration

Transfer of the step two pool to Phosphate buffered saline (PBS) or Tris buffered saline (TBS) was done by gel filtration on disposable PD-10 gel filtration columns (GE Healthcare cat. no. 17-0851- 01) according to the instructions of the manufacturer. PBS was prepared by 10-fold dilution of 10X PBS (Gibco cat. no. 70011) with water for injection (WFI, Fresenius Kabi cat. no. 883314). TBS was prepared by dissolving one tablet (Sigma Aldrich cat. no. 94158) in 500 ml of WFI. The TBS preparation was sterile filtered through a 0.22-µm filter. To avoid any contamination with endotoxin, single-use non-pyrogenic bottles (Corning) were used for preparation of PBS and TBS.

For PBS 19 aliquots of 2.5 ml were desalted on 19 PD-10 columns (total volume desalted to PBS 47.5 ml). Similarly, for TBS three aliquots of 2.5 ml were desalted on three PD-10 columns (total volume desalted to TBS 7.5 ml). The desalted products were stored at - 20°C.

The desalted products, labelled PSM 1646 (product in TBS) and PSM 1647 (product in PBS) were analyzed by:
1. SDS-PAGE two-fold dilutions, to estimate NN_GBS purity.
2. Total protein determination by Bradford protein assay.
3. Analytical size exclusion chromatography (SEC).
4. Endotoxin assay.

SDS-PAGE gels of the final product, buffer exchanged to PBS, repeatedly showed a smear of staining around the NN_GBS band, especially in lanes with a large amount of sample loaded. The smear was seen on both Coomassie blue and silver stained gels, as shown below. Strict use of fresh gels and reagents did not solve the problem, which was specifically seen on NN_GBS gels.

Therefore, as an alternative to the gel system Novex NuPAGE 12% homogeneous gels with MOPS running buffer, a "classic" Tris-glycine gel was run and Coomassie stained, Scanning of the gel with a Gel Doc EZ Imager (Bio-Rad) showed a purity of >95%.

Bradford protein assay: The final yield of NN_GBS after desalting, together with the yields after step one and two are shown in Table 6. The accumulated yield was 26%, i.e. for every 100 mg of total *E. coli* protein used for the purification, 26 mg of 95% pure NN_GBS was obtained. The overall recovery of NN_GBS was estimated to 60%.

Analytical SEC of the final product, buffer exchanged to PBS, showed a purity of >98%. The analysis indicate only minor impurities present in the final product. (Data not shown.)

The endotoxin concentration of the final products, in PBS and in TBS was less than 2 EU/mg. This result indicated that the low level of endotoxin found after step one, 3 EU/mg, was maintained or slightly improved in step two. These data indicate that the developed purification process efficiently reduced the endotoxin concentration to a very low level which is compatible with future in vivo use of the product.

**Table 6: SDS-PAGE and Bradford analysis. Two step purification of NN-GBS from a crude E. coli extract.**

| | Total protein Conc. (1) mg/ml | Volume ml | Amount of protein (2) mg | Yield accum. (3) % | Purity SDS-PAGE % | Purity analytical SEC (4) % | Accum. Recover y of NN-GBS (5) % |
|---|---|---|---|---|---|---|---|
| Crude extract loaded in step one | 20.48 | 25.00 | 512 | 100 | 42 | - | 100 |
| Pool after step one | 8.22 | 27.66 | 227 | 44 | 82 | - | 87 |
| Pool after step two | 2.34 | 57.62 | 135 | 26 | >95 | - | 60 |
| Product in PBS | 1.75 | 65.13 | 114 | - | - | >98 | - |
| Product in TBS | 1.75 | 10.44 | 18 | - | - | - | - |

| | | | | | | | |
|---|---|---|---|---|---|---|---|
| (1) Determined by Bradford protein assay using bovine gamma globulin as a standard (Bio-Rad). All samples were analyzed the same day using the same standard curve. (2) Calculation from Bradford assay result and volume. (3). Based on the protein amounts measured by Bradford assay. (4) the same purity was observed for the 214-nm and the 280-nm profile. (5) based on Bradford protein assay and purity estimated by SDS-PAGE. | | | | | | | |

### Calculation of concentrations and yields:

SDS-PAGE analysis of BugBuster lysates indicated the presence of 3.6 g/L NN_GBS (Bradford estimate) or 1.3 g/L (amino acid analysis estimate) in the fermentation product. These numbers were estimated by direct comparison with purified NN-GBS.

The concentration of NN_GBS in the fermentation product may also be estimated from the Bradford determination of total protein in the cell extract and from the purity determined using SDS-PAGE: Concentration in the fermentation product = [Protein concentration] x [volume of cell extract] x [Purity]/[Fermentor volume] = 20.48 mg/ml x 730 ml x 0.42/0.8 L = 7.8 g/L. This number is probably an overestimate, since Bradford analysis previously was shown to overestimate the NN_GBS concentration when compared to amino acid analysis, likely due to unusually strong Coomassie binding. This would mean that both the total protein concentration in the cell extract and the purity are overestimated, leading to an overestimate of the NN_GBS concentration in the fermentation product.

The yield of purified NN_GBS product from 25 ml (extract) = [Protein concentration] x [volume of product] x [Purity] = 2.34 mg/ml x 57.52 ml x 0.95 = 128 mg.

The total volume of cell extract from 0.8 L fermentor volume is 730 ml. Therefore, 25 ml of cell extract is equal to: 25 ml/730 ml x 800 ml = 27 ml fermentor volume.

The expected amount of purified NN_GBS per liter of fermentor culture is therefore= 128 mg/27 ml x 1000 ml = 4.7 g (Bradford) or 1.7 g (amino acid analysis).

### Summary of examples 1-3

A simple, two-step purification protocol for untagged NN_GBS, previously developed in low milligram scale, could be up-scaled to ~100 mg scale.

### In step one, crude E. coli extract, obtained by high-pressure homogenization, was fractionated on a DEAE Sepharose FF column. The purity of this intermediate product was ~82%.

In step two the product from step one was further purified by hydrophobic interaction chromatography on a Phenyl Sepharose FF column. The purity of the final product was >95% (SDS-PAGE, scanning of the gel) and >98% (analytical SEC).

The recovery of NN_GBS was estimated ~87% in step one, and the overall recovery (steps 1 and 2) was 60%.

Yield of purified NN_GBS: 512 mg of total protein in the crude extract used for the purification resulted in 135 mg of final product, corresponding to an overall yield of 26%.

Calculations showed that approx. 4.7 g (Bradford) or 1.7 g (amino acid analysis) of purified NN_GBS can be obtained from 1 liter of fermentation product.

Endotoxin level was reduced from ~300,000 EU/mg in the crude extract to <2 EU/mg in the final product, corresponding to a reduction of ~150,000 fold.

### EXAMPLE 4: Development of purification process suitable for large scale production of recombinant fusion proteins GBS-NN and GBS-NN2

### GBS-NN

### Step 1: Anion exchange chromatography (AIEX)

The start material for the purification is GBS-NN homogenate prepared by fermentation of *E. coli* expressing the GBS-NN recombinant fusion protein, harvesting, concentrating and washing the cells by tangential flow filtration and disrupting the cells. One bag (1.6 kg) of homogenate was thawed at +2 - +8 °C for 64 h, and 1300 ml was used for the purification comprising AIEX and HIC chromatography.

The homogenate (1300 ml, 6.4 mg/ml) was conditioned by mixing with 145 ml of 0.5 M Bis-Tris-HCl pH 6.0 solution and filtered. The solution was completely clear after buffer addition and after filtration. The anion chromatography was performed according to the conditions set forth in table 7 and the results are shown in Fig. 6.

**Table 7: Anion exchange conditions.**

| | | | |
|---|---|---|---|
| Column | DEAE Sepharose FF 5/21 cm, CV = 412 ml | Flow | 120 cm/h |
| Buffers | A: 20 mM Bis-Tris-HCl, pH 6.0 | | |
| | B1: 20 mM Bis-Tris-HCl, 60 mM NaCl, pH 6.0 | | |
| | B2: 20 mM Bis-Tris-HCl, 170 mM NaCl, pH 6.0 | | |
| | B3: 20 mM Bis-Tris-HCl, 1 M NaCl, pH 6.0 | | |
| Sample load | Approx. 20 g total protein/L media, sample conditioning 9 parts homogenate (1300 ml) + 1 part 0.5 M Bis-Tris pH 6.0 (145 ml), filtration 0.22 µm. | | |
| Elution | Wash with 3 CV buffer A | | |
| | Step elution with buffers B1 (4 CV), B2 (4 CV), B3 (3 CV) | | |
| Pooling | Main peak from B2 elution pooled based on chromatogram (fractions 1A6 - 1B3), in total 600 ml, filtered 0.22 µm. | | |
| Samplin g | 8 x 1 ml (AlEX-Start and AlEX-END), stored -20°C | | |
| Storage | +2 - +8°C, overnight | | |

### Step 2: Hydrophobic interaction chromatography (HIC)

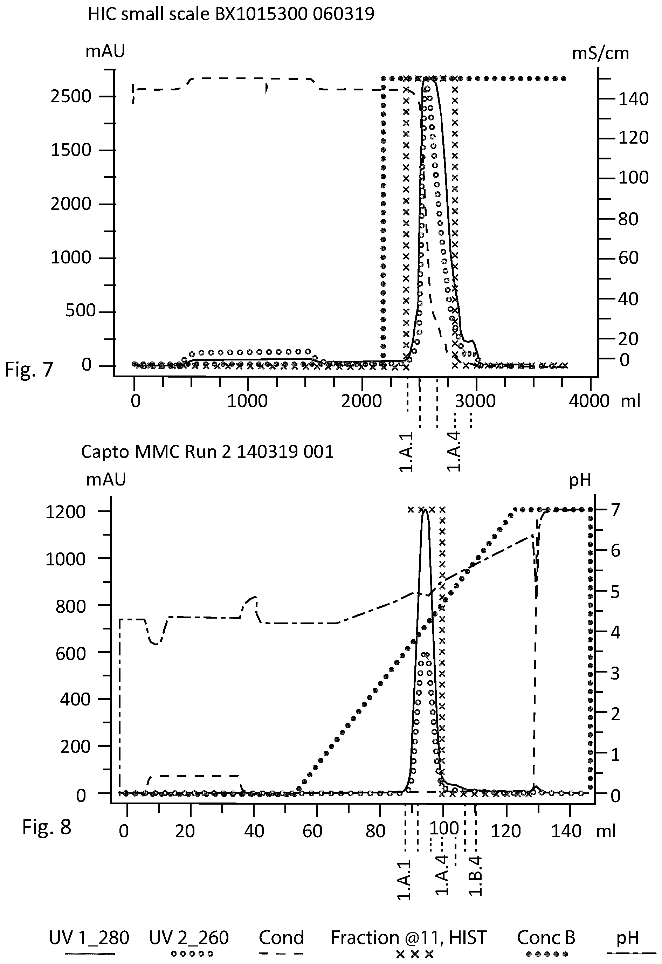

The AIEX-END (590 ml) was conditioned by mixing with 100 mM NaPO₄, 2 M ammonium sulfate, pH 7.0 solution, and filtered. The solution was completely clear after buffer addition and after filtration. The conditions for the hydrophobic interactions chromatography was as shown in table 8 and the results are shown in Fig. 7

**Table 8: HIC conditions**

| | | | |
|---|---|---|---|
| Column | Phenyl Sepharose 6 FF (Is) 5/21 cm, CV = 412 ml | Flow | 120 cm/h |
| Buffers | A: 50 mM NaPO4, pH 7.0, 1 M ammonium sulfate | | |
| | B: 50 mM NaPO4, pH 7.0 | | |
| Sample load | 17 g protein/L media, sample conditioning 1 part (590 ml) AlEX-END + 1 part (590 ml) 100 mM NaPO4, 2 M ammonium sulphate, pH 7.0, filtration 0.22 µm (Opticap XL150 SHC, PES). | | |
| Elution | Wash with 2.5 CV buffer A. | | |
| | Step elution with buffer B, 4 CV. | | |
| Pooling | Main peak pooled based on chromatogram (fractions 1A1 - 1A3) in total 410 ml. Filtered 0.22 µm and aliquoted (9x 41 ml, 1x 30 ml). | | |
| Samplin g | 8 x 1 ml (HIC-Start and HIC-END), stored -20°C | | |
| Storage | +2 - +8°C max. 4 weeks (1x 30 ml), rest (9x 41 ml) -20°C (no freeze-thaw). | | |

The HIC-END material was divided in 41 ml aliquots and stored in part at +2 - +8°C (39 ml, to be used for development work within 4 weeks) and rest (9 x41 ml) at -20°C.

Samples from GBS-NN HIC-END production were analyzed and the results are summarized in table 9.

**Table 9. Analysis results of samples from GBS-NN HIC-END material production. The concentration of homogenate was measured with Bradford method, other concentrations using UV280 method. For AIEX-Start the UV280 concentration is not accurate due to sample interference**

| **Sample** | **Homogenate** | **AIEX-Start** | **AIEX-END** | **HIC-Start** | **HIC-END** |
|---|---|---|---|---|---|
| Volume (ml) | 1300 | 1445 | 600 | 1180 | 410 |
| Concentration (mg/ml)* | 6.4 | 12.2 | 11.9 | 6.4 | 15.1 |
| Total protein (mg) | 8320 | 17629 | 7140 | 7552 | 6191 |
| Step yield (%) | 100 % | - | 86 % | - | 87 % |
| Total yield (%) | 100 % | 212 % | 86 % | 91 % | 74 % |
| HCP (ng/ml) ** | - | 11345 | 960 | 1080 | 509 |
| HCP (ng/mg) ** | - | 930 | 81 | 169 | 34 |
| Total HCP (µg) | - | 16394 | 576 | 1274 | 209 |
| HCP clearance | - | 0% | 96.5 % | 92.2 % | 98.7 % |
| hcDNA (ng/mg) ** | - | 1198 | 8.5 | 3.6 | 3.4 |
| Total hcDNA (µg) | - | 21122 | 61 | 27 | 21 |
| hcDNA clearance | - | 0% | 99.71 % | 99.87 % | 99.90 % |
| RP-HPLC purity | - | - | - | - | 96.6 % |
| SE-HPLC (purity, retention time, impurities % HMW area | - | - | - | - | 98.9 %, |
| Endotoxins (IU/mg) | - | - | - | - | < 0.33 |
| SDS-PAGE purity | - | Main band 35 kDa, 85.6 % | Main band 34 kDa, 94.9 % | Main band 35 kDa, 95.3 % | Main band 35 kDa, 96.0 % |

| | | | | | |
|---|---|---|---|---|---|
| .* Homogenate result from Bradford analysis, others from UV280. Interfering factors present in AIEX-Start sample. ** High CV in all samples, interfering factors present. | | | | | |

### Step 3: Finding an alternative to size exclusion chromatography

Size exclusion chromatography gave a satisfactory final product but was found to not be optimal for large scale production. Thus, this purification step had to be re-designed. It was thus tested whether size exclusion chromatography could be replaced with mixed mode chromatography.

For this purpose, three different types of mixed mode columns were tested, one for cation exchange, one for anion exchange and one cation exchange and metal affinity column. A summary of the characteristics of the three mixed mode columns are shown in table 10.

**Table 10. Features of mixed mode columns tested.**

| Resin | Manufacturer | Functional group | Base matrix |
|---|---|---|---|
| Capto MMC ImpRes *(MMC cation exchanger)* | GE Healthcare | Carboxyl (weak cation exchanger), amino, phenyl and thiol groups | High-flow agarose |
| Capto Adhere (MMC *anion exchanger)* | GE Healthcare | Quaternary ammonium (strong anion exchanger), phenyl and hydroxyl groups | High-flow agarose |
| Ceramic hydroxyapatite CHT type I (*MMC cation exchange and metal affinity)* | Bio-Rad | Calcium, phosphate and hydroxyl groups | Ceramic hydroxyap atite |

The start material for the test was the GBS-NN homogenate obtained after step 2 above. A test run was performed with each column using parameters defined based on previous development runs. Method performance and potential was assessed by comparing analysis results of pooled material from each run to results of start material. The experiment conditions for the MMC cation exchanger are shown in table 11.

**Table 11: MMC cation exchanger, Capto MMC ImpRes run 1 and run 2**

| | | | |
|---|---|---|---|
| Column | HiScreen Capto MMC ImpRes, 4.7 mL | Flow | 200 cm/h (1.55 mL/min) |
| Buffers | A: 20 mM Na-citrate, pH 4.5 | | |
| | B1: 20 mM Trisodium citrate (pH unadjusted) | | |
| | B2: 30 mM NaPO4, 2 M NaCl, pH 7.4 | | |
| Sample load | HIC-END, 10 mg/ml media (3.1 ml, 46.8 mg), | | |
| | sample diluted: Run 1: 1+3 with buffer A, filtered 0.22 µm, loaded 12 mL | | |
| | Run 2: 1+9 with buffer A, filtered 0.22 µm, loaded 30 mL | | |
| Elution | Gradient 0 - 100 % B1 over 15 CV, wash 5 CV B2 | | |
| Pooling | Run 1: no pooling. Flowthrough and fractions stored +2 - +8°C. | | |
| | Run 2: Main peak pooled based on UV280, fractions 1A1 - 1A3, in total 12 mL. Filtered 0.22 µm before sampling and storage (-20°C). | | |
| Sampling | Run 2: 8 x 1 ml, stored at -20°C | | |
| Analysis results | UV280 (Run 2 pool): 3.2 mg/ml Calculated step yield: 91 % | hcDNA (Run 2 pool): 4 ng/ml (1.3 ng/mg) hcDNA step clearance: 70 % | |

In Capto ImpRes Run 1 the sample leaked out during sample application due to a combination of pH fluctuation (sample buffer interaction with resin) and relatively high sample conductivity (sample diluted 1 + 3 with buffer A). Only a small peak was observed in elution phase. A new run (Run 2) was performed (Fig. 8), where sample was diluted more (1 + 9 with buffer A). This prevented sample leak, and the run was completed successfully.

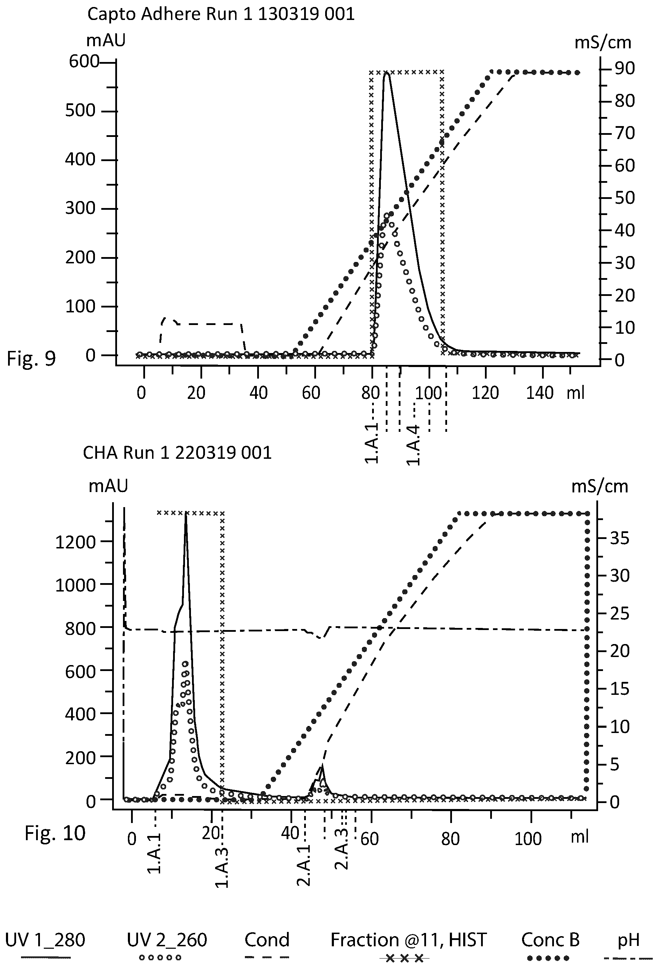

The MMC anion exchange was also tested. The results are shown in Fig. 9 and experiment conditions are shown in table 12.

**Table 12: MMC anion exchanger, Capto Adhere run 1.**

| | | | | |
|---|---|---|---|---|
| Column | HiScreen Capto Adhere, 4.7 mL | Flow | | 200 cm/h |
| Buffers | A: 20 mM Bis-Tris, pH 6.0 | | | |
| | B: 20 mM Bis-Tris, 1 M NaCl, pH 6.0 | | | |
| Sample load | HIC-END, 10 mg/ml media (3.1 ml, 46.8 mg), sample diluted 1+9 with buffer A, filtered 0.22 µm, loaded 29 mL | | | |
| Elution | Gradient 0 - 100 % B over 15 CV, wash 5 CV B | | | |
| Pooling | Main peak pooled based on UV280, fractions 1A1 - 1A5, in total 25 mL. Filtered 0.22 µm before sampling and storage (-20°C). | | | |
| Sampling | 8 x 1 ml, stored -20°C | | | |
| Analysis results | UV280 (Run 1 pool): 1.7 mg/ml Calculated step yield 82% | | hcDNA (Run 1 pool): 4 ng/ml (2.4 ng/mg) hcDNA step clearance: 37 % | |

The MMC cation exchange and metal affinity was also tested. The results are shown in Fig. 10 and experiment conditions are shown in table 13.

**Table 13: MMC cation exchange and metal affinity, Ceramic HA run 1.**

| | | | |
|---|---|---|---|
| Column | Bio-Scale Mini CHT Type I Cartridge, 5 mL | Flow | 200 cm/h |
| Buffers | A: 5 mM NaPO₄, pH 6.8 | | |
| | B: 500 mM NaPO₄, pH 6.8 | | |
| Sample load | HIC-END, 10 mg/ml media (49.8 mg). Sample (3.3 ml HIC-END) desalted to buffer A with two PD-10 columns and filtered 0.22 µm, loaded 6.5 ml. | | |
| Elution | Flowthrough-mode, flowthrough collected. Washed with 5 CV buffer A after sample load, gradient 0 - 100 % B over 10 CV, wash 5 CV B. | | |
| Pooling | Flowthrough pooled based on UV280 (fractions 1A1-1A2, in total 17 ml). | | |
| | Filtered 0.22 µm before sampling and storage (-20°C). | | |
| Sampling | 8 x 1 ml, stored -20°C | | |
| Analysis results | UV280 (Run 1 Start, desalted and filtered): 7.1 mg/mL UV280 (Run 1 FT-pool): 2.2 mg/mL | hcDNA (Run 1 FT-pool): 5 ng/ml (2.3 ng/mg) hcDNA step clearance: 49 % | |
| | Calculated step yields: sample pretreatment 100 %, CHA step 75 % | | |

*Results of comparison between the three tested mixed mode columns*

The result of the comparison between the different types of mixed mode columns are presented in Table 14. In comparison with SEC, all three mixed mode columns tested provided a higher yield of product (SEC yield 72 - 82 %, MMC yield 75 - 91 %). The best results were obtained with Capto Adhere (91 %). The HCP and hcDNA analysis results were not reliable most likely due to interfering factors in samples. Overall differences between the results of all three runs were small.

**Table 14: Summary of MMC test run analysis results and comparison to start material and Drug Substance (DS) from previous GMP batches. A calculated estimate of the total process yield if using MMC step as third process step is included.**

| Sample | HIC-END | Capto/ CHA start* | Capto MMC pool | Capto Adhere pool | CHA pool | Previous GMP batches (DS) |
|---|---|---|---|---|---|---|
| Volume (ml) | 410 | 3.1/ 3.3 | 12 | 25 | 17 | - |
| Concentration (mg/ml) | 15.1 | 15.1 | 3.2 | 1.7 | 2.2 | - |
| Total protein yield (mg) | 6191 | 46.8/49.8 | 38.4 | 42.5 | 37.4 | - |
| Step yield (%) | 87% | - | 82% | 91% | 75% | 72-82% (SEC) |
| Total yield (%) | 74% | - | Est. 61% | Est. 68% | Est. 56% | 45-56% |
| HCP (ng/ml)** | 509 | 509 | 121 | 148 | 116 | 80-297 |
| HCP (ng/mg)** | 34 | 34 | 38 | 87 | 53 | 74-176 |
| Total HCP (ug) | 209 | 1.58/ 1.68 | 1.45 | 3.70 | 1.97 | - |
| HCP clearance (%) | 98.7% | - | 8.0% | -134% | -17% | - |
| hcDNA (ng/ml)** | 51 | 51 | 4 | 4 | 5 | <3.1 pg/ml (qPCR) |
| hcDNA (ng/mg)** | 3.4 | 3.4 | 1.3 | 2.4 | 2.3 | - |
| Total hcDNA (ug) | 21 | 0.158/ 0.168 | 0.048 | 0.100 | 0.085 | - |
| hcDNA clearance | 99.90% | - | 70% | 37% | 49% | - |
| RP-HPLC purity | 96.6% | 96.6% | 88.4%*** | 97.1% | 97.0% | 70-88% |
| SE-HPLC (purity, | 98.9% | 98.9% | 99.9% | 99.5% | 99.8% | 90-94% |
| | 5.4 min | 5.4 min | 5.4 min | 5.4 min | 5.4 min | |

| retention time, impurities & HMW area) | 1.1% | 1.1% | 0.1% | 0.5% | 0.2% | |
|---|---|---|---|---|---|---|
| Endotoxins (IU/mg) | <0.33 | <0.33 | <1.6 | <2.9 | <2.3 | 0.3-3 IU/mg |
| SDS-PAGE purity | Main band 35 kDa 96.0% | Main band 35 kDa 96.0% | Main band 35 kDa 97.0% | Main band 35 kDa 98.2% | Main band 35 kDa 96.0% | >95% |

| | | | | | | |
|---|---|---|---|---|---|---|
| * 3.1 ml of HIC-END loaded on Capto MMC and Adhere, 3.3 ml on CHA. ** High CV in all samples, interfering factors present. *** RP-HPLC chromatogram profile of Capto MMC pool differs from other samples. | | | | | | |

As the criteria for method selection were increasing process robustness and improvements to product purity the anionic mixed mode column (Capto Adhere) was considered the most preferred column even if the overall difference between the different columns was small.

### GBS-NN2

### Step 1: Anion exchange chromatography (AIEX)

As the anion mixed mode column Capto Adhere was found to be the best choice for the third purification step to purify GBS-NN, it was further tested if this type of column was also suitable for purification of GBS-NN2 to ensure suitability for other recombinant fusion proteins but GBS-NN.

The GBS-NN2 test material was produced using AIEX and HIC columns (XK16 scale) with a lower resin bed height than used for feasibility scale column packing. Purification run flow rate was adjusted to 48 cm/h to keep sample residence time same as in feasibility scale.

One bottle (313 g) of GBS-NN2 homogenate (produced in a similar fermentation process as described above for GBS-NN) was thawed first at room temperature (2 h), then overnight at +2 - +8 °C for 16 h, and 125 ml was used for the small-scale test material purification.

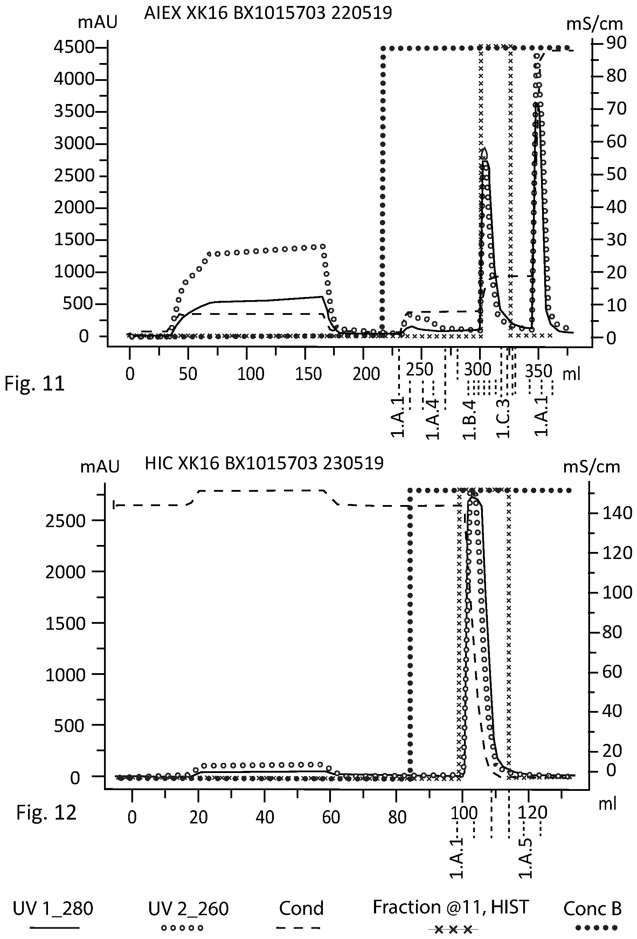

The homogenate (125 ml, 2.4 mg/ml) was conditioned by mixing with 14 ml of 0.5 M Bis-Tris-HCl pH 6.0 solution and filtered. The solution was completely clear after buffer addition and after filtration. Sampling (4x1 ml) was performed from both step start and step end material. The elution profile of this run (Fig. 11) was similar to GBS-NN small scale AIEX run (section 1.1, Fig. 6). Experiment conditions are shown in table 15 for the anion exchange chromatography.

**Table 15: Anion exchange conditions.**

| | | | |
|---|---|---|---|
| Column | DEAE Sepharose FF 1.6/7.5 cm, CV = 15 mL | Flow | 48 cm/h (residence time 10 min) |
| Buffers | A: 20 mM Bis-Tris-HCl, pH 6.0 | | |
| | B1: 20 mM Bis-Tris-HCl, 60 mM NaCl, pH 6.0 | | |
| | B2: 20 mM Bis-Tris-HCl, 170 mM NaCl, pH 6.0 | | |
| | B3: 20 mM Bis-Tris-HCl, 1 M NaCl, pH 6.0 | | |
| Sample load | Approx. 20 g total protein/L media, sample conditioning 9 parts homogenate (125 ml) + 1 part 0.5 M Bis-Tris pH 6.0 (14 ml), filtration 0.22 µm. | | |
| Elution | Wash with 4 CV buffer A | | |
| | Step elution with buffers B1 (5 CV), B2 (3 CV), B3 (3 CV) | | |
| Pooling | Main peak from B2 elution pooled based on chromatogram (fractions 1B4 - 1C3), in total 25 ml, filtered 0.22 µm. | | |
| Sampling | 4 x 1 ml (AIEX-Start) and 4 x 0.5 ml (AIEX-END), stored -20°C | | |
| Storage | +2 - +8°C, overnight | | |
| Analysis results | UV280 (AlEX-Start): 13.9 | | |
| | g/ml UV280 (AIEX.END): 12.9 mg/ml | | |
| | Calculated step yield: 108 % (of start amount of Homogenate) | | |

### Step 2: Hydrophobic interaction chromatography (HIC)

The AlEX-END (23 ml) was conditioned by mixing with 100 mM NaPO₄, 2 M ammonium sulfate, pH 7.0 solution, and filtered. The solution was completely clear after buffer addition and after filtration. Sampling (4 x 0.5 ml) was performed from both step start and step end material. Experiment conditions for the HIC chromatography are shown in table 16.

**Table 16: HIC chromatography conditions**

| | | | |
|---|---|---|---|
| Column | Phenyl Sepharose 6 FF (Is) 1.6/8 cm, CV = 16 mL | Flow | 48 cm/h (residence time 10 min) |
| Buffers | A: 50 mM NaPO₄, pH 7.0, 1 M ammonium sulfate | | |
| | B: 50 mM NaPO₄, pH 7.0 | | |
| Sample load | 19 g protein/L media, sample conditioning 1 part (23 ml) AlEX-END + 1 part (23 ml) 100 mM NaPO₄, 2 M ammonium sulfate, pH 7.0, filtration 0.22 µm. | | |
| Elution | Wash with 2.6 CV buffer A. Step elution with buffer B, 3 CV. | | |
| Pooling | Main peak pooled based on chromatogram (fractions 1A1 - 1A3) in total 15 ml. Filtered 0.22 µm and aliquoted (6x 1.5 ml, 1x 0.5 ml, 1x 3 ml). | | |
| Sampling | 4 x 0.5 ml (HIC-Start and HIC-END), stored -20°C | | |
| Storage | +2 - +8°C max. 4 weeks (3 ml), rest -20°C (no freeze-thaw). | | |
| Analysis results | UV280 (HIC-Start): 6.5 mg/ml | | |
| | UV280 (HIC-END): 17.1 m mg/ml | | |
| | Calculated step yield: 86 % (from AIEX-END) | | |

The elution profile from this experiment (Fig. 12) was similar to GBS-NN small scale HIC run (section 1.1, Fig. 7). The HIC-END material was divided in aliquots and stored in part at +2 - +8°C (3 ml, to be used for development work within 4 weeks) and rest (6x 1.5 ml, 1x 0.5 ml) at -20°C.

### Step 3: Mixed mode column Capto Adhere (polishing step)

The GBS-NN2 HIC-END material (from step 2) was used in small scale to test Capto Adhere performance with GBS-NN2, experiment conditions are shown in table 17. The run parameters were same as in Capto Adhere GBS-NN Run 1 (see 1.1, step 3) to enable direct comparison of the runs.

**Table 17: Capto Adhere conditions.**

| | | | |
|---|---|---|---|
| Column | HiScreen Capto Adhere, 4.7 mL | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN2 HIC-END, 10 mg/ml media (47 mg), sample diluted 1+9 with buffer A, filtered 0.22 µm, loaded 27 mL | | |
| Elution | Gradient 0 - 100 % B over 15 CV, wash 5 CV B | | |
| Pooling | Main peak pooled based on UV280, fractions 1A1 - 1B1, in total 30 mL. | | |
| | Filtered 0.22 µm before sampling and storage (-20°C). | | |
| Sampling | 8 x 1 ml, stored -20°C | | |
| Analysis results | UV280 (Run 1 pool): 1.26 mg/ml Calculated step yield 81% | | |

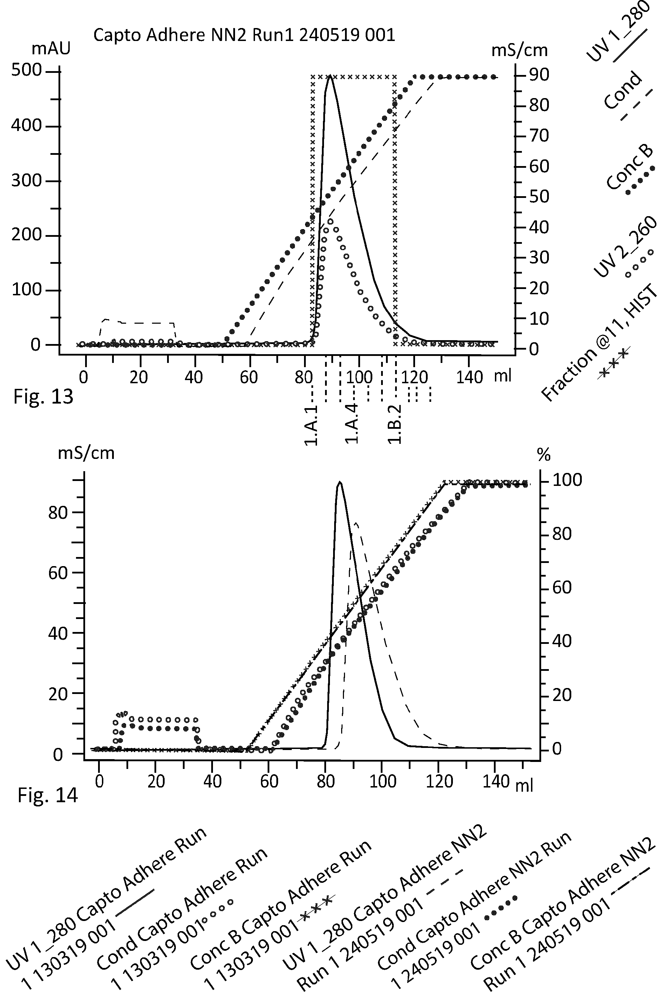

Compared to GBS-NN Capto Adhere run (see 1.1 step 3 above), the GBS-NN2 elution peak is wider and elutes at slightly higher conductivity (Fig. 13). For ease of comparison between Capto adhere runs of GBS-NN and GBS-NN2 see Fig. 14 which is an overlay of both experiments chromatogram. Based on the chromatograms the Capto Adhere purification appears to function equally for GBS-NN and GBS-NN2. The yield was slightly lower for GBS-NN2 (81 %) compared to GBS-NN (91 %).

As a summary of Example 4, it was found that there is a need for further optimizing of the anion mixed mode chromatography step

### EXAMPLE 5: Optimization of anion mixed mode chromatography

In this example the anion mixed mode column step was further developed and optimized (determination of suitable start material dilution, development of step elution parameters, flow rate and resin capacity testing). Both GBS-NN and GBS-NN2 material was used in tests.

### GBS-NN

### Step elution testing

Start parameters for step elution were chosen based on GBS-NN and GBS-NN2 product peak elution during gradient elution in previous tests. In this first test, sample dilution in 1+4 ratio (sample + buffer A) was tested, experimental conditions are shown in table 18.

**Table 18: Conditions GBS-NN, Capto Adhere Run 2 (step elution test 1).**

| | | | |
|---|---|---|---|
| Column | HiScreen Capto Adhere, 4.7 ml | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, 10 mg/ml media (47 mg), sample diluted 1+4 with buffer A, filtered 0.22 µm, loaded 14 ml | | |
| Elution | Wash 5 CV A | | |
| | Step 1: 25% B, 3 CV | | |
| | Step 2: 60% B, 3 CV, fractions 5 ml | | |
| | Step 3: 100% B, 3 CV | | |
| Pooling | No pooling, fractions stored (+2 - +8°C) | | |

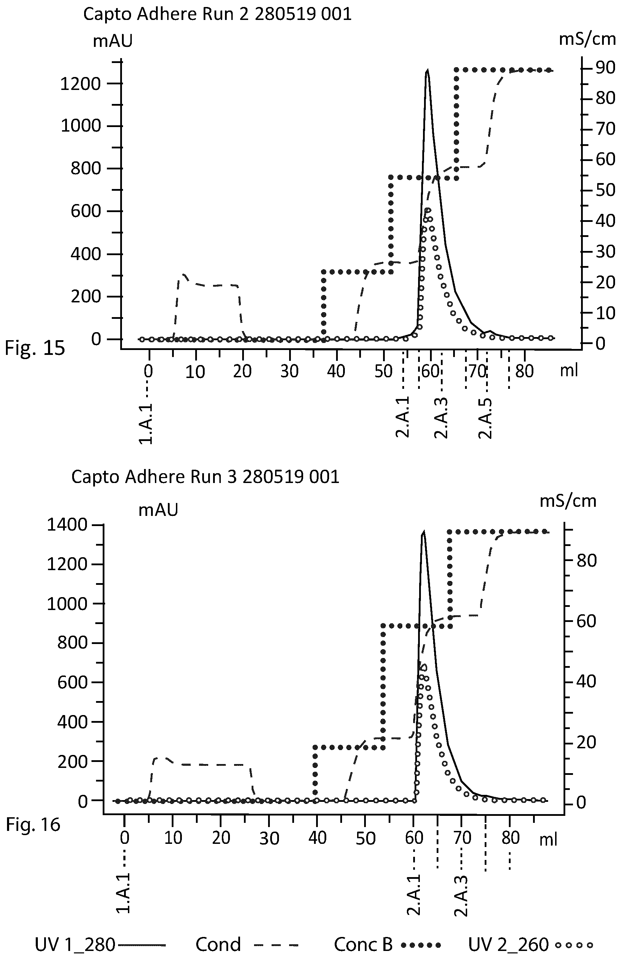

The experiment showed that 1+4 dilution of start material is enough for efficient binding (Fig. 15). There was no detectable impurity peak in Step 1 elution (25% B), but product peak started to leak out at end of this step suggesting lower percentage of buffer B in Step 1 elution would be better. Elution of peak in Step 2 (60% B) was good, but slightly higher percentage of buffer B would elute the product as a more compact peak. It was decided to test higher dilution of start material, lower Elution Step 1 percentage and higher Elution Step 2 percentage in next run.

In the next experiment, sample dilution in 1+7 ratio (sample + buffer A) was tested, experimental conditions are shown in table 19.

**Table 19: Conditions GBS-NN, Capto Adhere Run 3 (step elution test 2).**

| | | | |
|---|---|---|---|
| Column | HiScreen Capto Adhere, 4.7 mL | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, 10 mg/ml media (47 mg), sample diluted 1+7 with buffer A, filtered 0.22 µm, loaded 21 ml | | |
| Elution | Wash 4 CV A | | |
| | Step 1: 20% B, 3 CV | | |
| | Step 2: 65% B, 3 CV, fractions 5 ml | | |
| | Step 3: 100% B, 3 CV | | |
| Pooling | No pooling, fractions stored (+2 - +8°C) | | |

This experiment (Run 3) showed that 1+7 dilution of start material works equally well (Fig. 16). There was no impurity peak or product leakage in elution Step 1 when using 20% B, and the product eluted as a sharper, more compact peak during elution Step 2 (now 65% B). It was thus decided to test if the parameters of Run 3 also work for GBS-NN2 material.

### GBS-NN2

The previous experiment was repeated with GBS-NN2 at a sample dilution of 1+4 ratio (sample + buffer A), experimental conditions are shown in table 20.

**Table 20: GBS-NN2, Capto Adhere Run 2 (step elution test 1 of GBS-NN2).**

| | | | |
|---|---|---|---|
| Column | HiScreen Capto Adhere, 4.7 mL | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN2 HIC-END, 10 mg/ml media (47 mg), sample diluted 1+4 with buffer A, filtered 0.22 µm, loaded 13 ml | | |
| Elution | Wash 4 CV A | | |
| | Step 1: 20% B, 3 CV | | |
| | Step 2: 65% B, 4 CV, fractions 5 ml | | |
| | Step 3: 100% B, 3 CV | | |
| Pooling | No pooling, fractions stored (+2 - +8°C) | | |

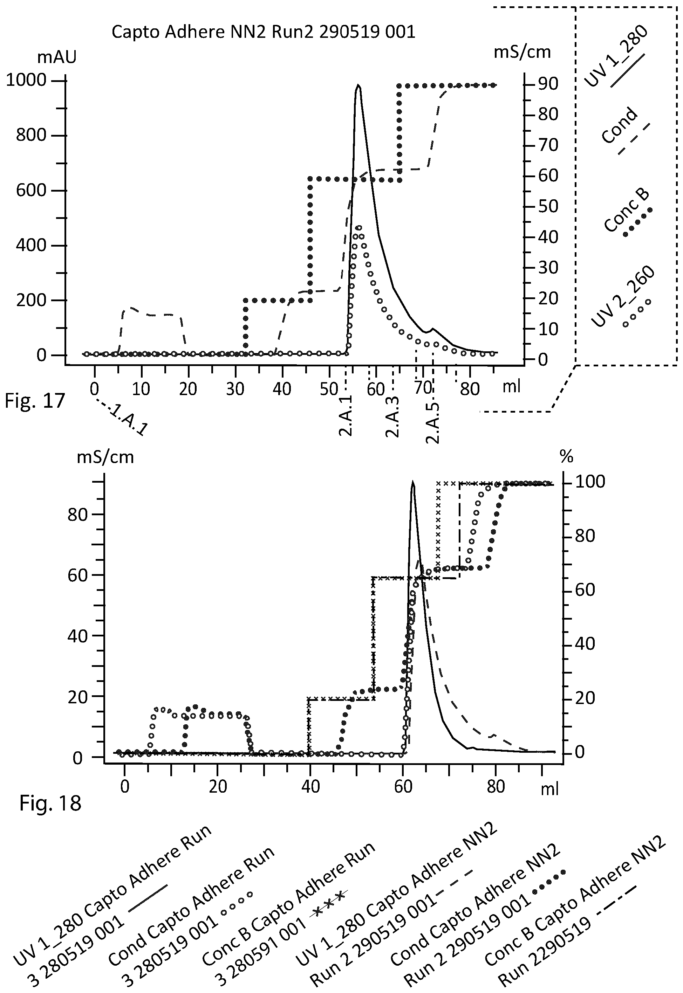

The chromatogram from this experiment is shown in Fig. 17 and it was deemed that for GBS-NN2 the step elution parameters needed to be optimized. GBS-NN2 requires either higher percentage of B or a longer step to elute completely (here 4 CV elution was used instead of 3 CV as for GBS-NN, but this was still insufficient). Dilution of start material works similarly to GBS-NN (dilution 1+4 works well). Comparison of the elution of both proteins using same elution steps is presented in Fig. 18.

### EXAMPLE 6: Optimization of column bed height, elution step and flow rate for polishing step (step iii).

### GBS-NN

### Capto Adhere Run 4 (bed height 20 cm)

In Run 4, two HiScreen Capto Adhere columns were used in series to mimic production scale bed height (20 cm). Start material 1+7 dilution was used to achieve good binding. Elution step length was increased to 4 CV and elution step buffer B concentration to 70 % to completely elute the product, experiment conditions are summarized in table 21.

**Table 21. Conditions GBS-NN, Capto Adhere Run 4 (bed height 20 cm)**

| | | | |
|---|---|---|---|
| Column | HiScreen Capto Adhere, 2x 4.7 ml (bed height 20 cm) | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, 10 mg/mL media (94 mg), sample diluted 1+7 with buffer A, filtered 0.22 µm, loaded 50 ml | | |
| Elution | Wash 4 CV A | | |
| | Step 1: 20% B, 4 CV | | |
| | Step 2: 70% B, 8 CV, fractions 5 mL | | |
| | Step 3: 100% B, 3 CV | | |
| Pooling | No pooling, fractions stored (+2 - +8°C) | | |
| Sampling | 1 ml and 0.2 ml per elution fraction | | |

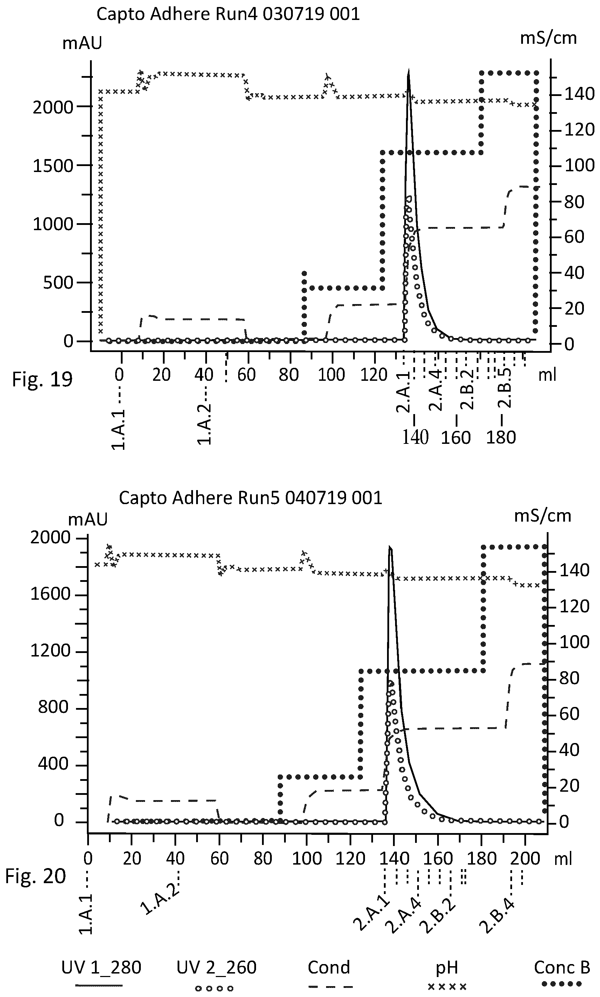

The peak eluted nicely on the higher column, and the peak was compact (Fig. 19). However, based on SDS-PAGE analysis of Run 4 elution peak fractions (data not shown) it is suspected that high MW contaminants coelute in the tail of the elution peak. In this light it would be better to make the elution peak wider to improve separation of the contaminants.

### GBS-NN

### Capto Adhere Run 5 (elution step optimization)

In Run 5 wash and elution step %B were adjusted to 17 % and 55% B to keep product tightly bound during wash step and to better separate the product and possible contaminants in elution step (step length increased to compensate slower elution). Experiment conditions are shown in table 22, results are shown in Fig. 20.

**Table 22: Conditions GBS-NN, Capto Adhere Run 5.**

| | | | |
|---|---|---|---|
| Column | HiScreen Capto Adhere, 2x 4.7 ml (bed height 20 cm) | Flow | 200 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, 10 mg/ml media (94 mg), sample diluted 1+7 with buffer A, filtered 0.22 µm, loaded 50 ml | | |
| Elution | Wash 4 CV A | | |
| | Step 1: 17% B, 4 CV | | |
| | Step 2: 55% B, 8 CV, fractions 5 mL | | |
| | Step 3: 100% B, 3 CV | | |
| Pooling | No pooling, fractions stored (+2 - +8°C) | | |
| Sampling | 1 ml and 0.2 ml per elution fraction | | |

Based on SDS-PAGE results, using 55 %B at Step 2 elution may slightly improve separation of GBS-NN from contaminants eluting in peak tail. It should be noted that these conditions are most likely not suitable for eluting GBS-NN2.

### GBS-NN

### Capto Adhere Run 6 (flow rate increase)

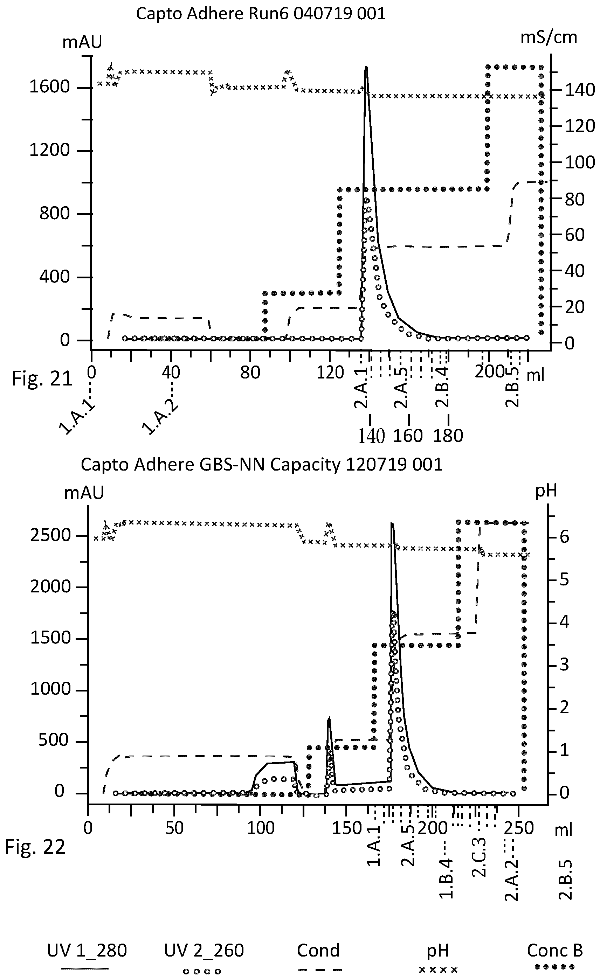

In Run 6 the run flow rate was increased to 300 cm/h to decrease sample residence time from 6 min to 4 min, experiment conditions are shown in table 23. The elution profile using 300 cm/h flow rate was practically identical to previous runs (200 cm/h), suggesting increasing flow rate to 300 cm/h is acceptable (Fig. 21).

**Table 23. Conditions GBS-NN, Capto Adhere Run 6 (flow rate increase).**

| | | | |
|---|---|---|---|
| Column | HiScreen Capto Adhere, 2x 4.7 ml (bed height 20 cm) | Flow | 300 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, 10 mg/mL media (94 mg), sample diluted 1+7 with buffer A, filtered 0.22 µm, loaded 50 ml | | |
| Elution | Wash 4 CV A | | |
| | Step 1: 17% B, 4 CV | | |
| | Step 2: 55% B, 8 CV, fractions 5 mL | | |
| | Step 3: 100% B, 3 CV | | |
| Pooling | No pooling, fractions stored (+2 - | +8°C) | |
| Sampling | 1 ml and 0.2 ml per elution fraction | | |
| Analysi s results | No analyses | | |

### EXAMPLE 7: Load range determination for the polishing step (step iii)

### GBS-NN

### Capto Adhere Capacity run

Capacity of the Capto Adhere resin for product binding was tested. Two columns were used for a combined bed height of 20 cm and flow rate was 300 cm/h. Starting material was loaded until complete product breakthrough was seen in flowthrough UV280. Experiment conditions are shown in table 24.

**Table 24: GBS-NN, Capto Adhere Capacity run.**

| | | | |
|---|---|---|---|
| Column | HiScreen Capto Adhere, 2x 4.7 mL | Flow | 300 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, sample diluted 1+7 with buffer A, filtered 0.22 µm, loaded until FT breakthrough. FT collected as one fraction. | | |
| Elution | Wash 2 CV A | | |
| | Step 1: 17% B, 4 CV | | |
| | Step 2: 55% B, 8 CV, fractions 5 mL | | |
| | Step 3: 100% B, 3 CV | | |
| Pooling | Step 2 fractions 1A2 - 1B4 (40 ml), | | |
| | Wash peak 1C3 - 1C4 (10 ml); stored +2 - +8°C | | |
| Sampling | No sampling | | |
| Analysis results | UV280: Start 2.2 mg/ml, Step 2 pool 2.9 mg/ml | | |

In total 108 ml (238 mg) of start material was loaded. 10% sample breakthrough was seen at 94.6 ml load point, where 84.3 ml = 185 mg = 20 g protein/L resin had run through column (Fig. 22). If column maximum load is defined at 10% sample breakthrough (20 g/L resin), then an estimated safe load level would be ≤ 80% of maximum load, i.e. ≤ 16 g/L resin.

An elution peak appeared in Step 1 (17% B, see Table 24 above) elution, accompanied by a drift of the UV signal to 100 mAU after the peak. Neither of these had been observed in any previous runs. Step 1 peak and UV drift are most likely due to column overload and subsequent slow release of weakly bound protein. The step 2 elution peak pool contains approximately 120 mg protein (yield approx. 65 % of load at 10% breakthrough).

### GBS-NN

### Capto Adhere Run 7 (load test)

A test run was done using same parameters as in capacity test except for a load volume of 80 ml (176 mg = 19 g/L resin, 95% of the maximum (10% breakthrough) load amount). Also, the length of the wash step after loading was increased to wash out any weakly bound material. Experiment conditions are summarized in table 25.

**Table 25: GBS-NN, Capto Adhere Run 7 (load test).**

| | | | |
|---|---|---|---|
| Column | HiScreen Capto Adhere, 2x 4.7 mL | Flow | 300 cm/h |
| Buffers | A: 20 mM Bis-Tris, pH 6.0 | | |
| | B: 20 mM Bis-Tris, 1 M NaCl, pH 6.0 | | |
| Sample load | GBS-NN HIC-END, sample diluted 1+7 with buffer A, filtered 0.22 µm, 19 mg/ml media (80 ml, 176 mg) loaded. FT collected as one fraction. | | |
| Elution | Wash 4 CV A | | |
| | Step 1: 17% B, 4 CV | | |
| | Step 2: 55% B, 8 CV, fractions 5 mL | | |
| | Step 3: 100% B, 3 CV | | |
| Pooling | Step 2 fractions 3A5 - 3C2 (40 ml), | | |
| | Wash peak 3C4 - 3C5 (10 ml); fractions 3A1 - 3A3 stored separately; | | |
| | Storage +2 - +8°C | | |
| Sampling | 1 + 0.2 ml per pool or fraction | | |
| Analysi s results | UV280: Start 2.2 mg/ml, Step 2 pool 3.5 | | |
| | mg/ml Calculated yield 68 % | | |

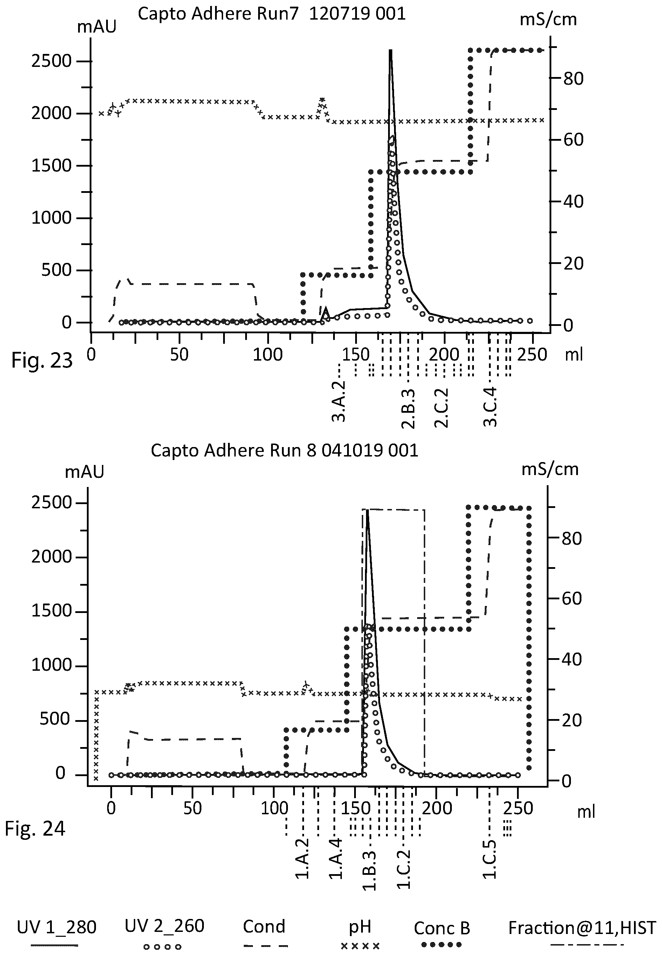

There was no product breakthrough during sample loading, but in Step 1 wash a small elution peak and UV signal drift to 100 mAU were seen, similarly to capacity run (Fig. 23). This suggests the used load (19 mg/ml resin) was slightly too high causing the product to leak out already during Step 1 wash. Step 2 elution peak pool contains approx. 120 mg protein, giving a yield of 68 %.

### GBS-NN

### Capto Adhere Run 8 (load test)

The aim of Run 8 was to produce more starting material for formulation step development. At the same time, the run was used to further test suitable Capto Adhere load amount. The used column load was 15 mg/mL resin, 75% of the maximum load (20 g/L) at 10% breakthrough and 94 % of the calculated maximum safe load (16 mg/ml resin). Experiment conditions are summarized in table 26.

**Table 26: GBS-NN, Capto Adhere Run 8 (load test).**

| | | | | |
|---|---|---|---|---|
| Column | HiScreen Capto Adhere, 2x 4.7 ml | Flow | | 300 cm/h |
| Buffers | A: 20 mM Bis-Tris, pH 6.0 | | B: 20 mM Bis-Tris, 1 M NaCl, pH 6.0 | |
| Sample load | GBS-NN HIC-END, sample diluted 1+7 with buffer A, filtered 0.22 µm, 15 mg/ml media (70 ml, 140 mg) loaded. FT collected as one fraction. | | | |
| Elution | Wash 4 CV A | | | |
| | Step 1: 17% B, 4 CV | | | |
| | Step 2: 55% B, 8 CV, fractions 5 mL | | | |
| | Step 3: 100% B, 3 CV | | | |
| Pooling | Step 2 fractions 3A5 - 3C2 (40 ml), | | | |
| | Wash peak 3C4 - 3C5 (10 ml); fractions 3A1 - 3A3 stored separately; Storage +2 - +8°C | | | |
| Sampling | 1 + 0.2 ml per pool or fraction | | | |
| Analysis results | UV280: Start 2.0 mg/ml, Step 2 pool | | | |
| | 3.6 mg/ml Calculated yield 99 % | | | |

Neither product breakthrough nor any elution peak or UV drift in Step 1 elution was observed (Fig. 24). The product eluted nicely in Step 2 (see Table 26 above). The used load amount appeared suitable, and yield was good (99 %).

### GBS-NN

### Capto Adhere Run 9 and 10 (material production, load test)

The aim of Runs 9 and 10 was to produce more material for formulation step development. At the same time, the runs were used to further test Capto Adhere load amount. The used column load was approx. 13 mg/ml resin (Run 9) and approx. 16 mg/ml resin (Run 10), 65% and 80% of the maximum load (20 g/l) at 10% breakthrough or 80 % and 100 % of the calculated maximum safe load (16 mg/ml resin). Experiment conditions are summarized in table 27.

**Table 27: Conditions GBS-NN, Capto Adhere Run 9 and 10 (material production, load test).**

| | | | | |
|---|---|---|---|---|
| Column | HiScreen Capto Adhere, 2x 4.7 ml | Flow | | 300 cm/h |
| Buffers | A: 20 mM Bis-Tris, pH 6.0 | | B: 20 mM Bis-Tris, 1 M NaCl, pH 6.0 | |
| Sample load | GBS-NN HIC-END, sample diluted 1+7 with buffer A, filtered 0.22 µm, Run 9: 13 mg/ml media loaded (60 ml, 120 mg). FT collected as one fraction. Run 10: 16 mg/ml media loaded (74 ml, 148 mg). FT collected as one fraction. | | | |
| Elution | Wash 4 CV A | | | |
| | Step 1: 17% B, 4 CV | | | |
| | Step 2: 55% B, 8 CV, fractions 5 ml | | | |
| | Step 3: 100% B, 5 CV | | | |
| Pooling | Step 2 fractions 1B2 - 1C4 (40 ml) in each run; Storage +2 - +8°C | | | |
| Sampling | Start material 2 ml, Filtered pool 0.5 ml per run, storage -20 °C | | | |
| Analysi s results | UV280: Start 2.0 mg/ml, Run 9 Step 2 pool 2.9 mg/ml, Run 10 Step 2 pool 3.5 mg/ml. Calculated yield Run 9: 94 %, Run 10: 93 %. | | | |

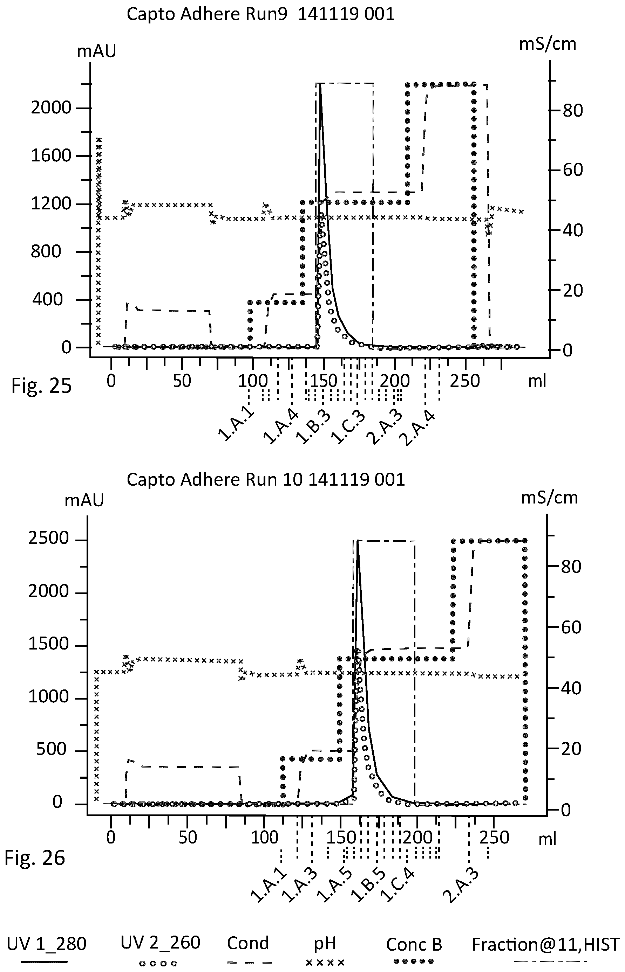

There was no product breakthrough in either Run 9 (Fig. 25) or Run 10 (Fig. 26) during load or column wash. In Run 10 with 16 mg/ml resin load the product started to leak out immediately before Step 2 elution, indicating that the 16 mg/ml resin load is the upper limit of safe load. The leaked product was not included in the elution pool. Despite the different load and product leakage the yield was almost identical in these two runs (94 % and 93 %).

In summary, the above demonstrates that the purification method can be optimized by adjusting e.g. bed height, flow rate, sample load and elution conditions.

### EXAMPLE 8: Development of formulation step (step iv))

The aim of this example is to prepare a purified homogenate suitable for formulation into a pharmaceutical product. To this end, a buffer exchange into formulation buffer after the MMC chromatography is necessary. The alternative methods considered were desalting chromatography, dialysis or diafiltration.

### Tangential flow filtration (TFF) tests

First tests were made using diafiltration by tangential flow filtration (TFF). Start material for formulation step experiments was various Capto Adhere pools from 3rd step development runs (see above). Experiment conditions are shown in table 28.

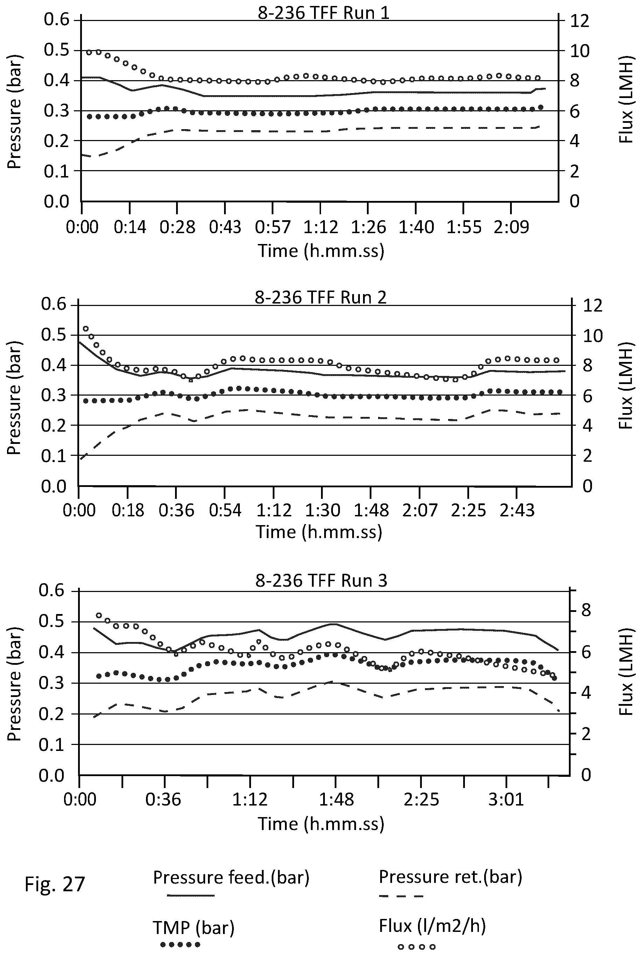

Three TFF runs (Fig. 27) were performed using pooled material from Capto Adhere runs. The used TFF membrane cassette was Repligen TangenX Pro PD HyStream (mPES membrane), 5 kDa, LP screen, 100 cm². In each run a different buffer was used for diafiltration (a phosphate buffer, a Tris/NaCl buffer, or a Histidine/sorbitol buffer, see process parameters in Table 28 below).

**Table 28: Conditions TFF tests.**

| | TFF Run 1 | TFF Run 2 | TFF Run 3 |
|---|---|---|---|
| Start material | GBS-NN Capto Adhere run 2 (2A2 - 2A3) and run 3 (2A1 - 2A3) | GBS-NN Capto Adhere run 7 (3A5 - 3C2) | GBS-NN Capto Adhere Capacity run (1A2 - 1B4) |
| Membrane | TangenX Pro PD HyStream 5 kDa | TangenX Pro PD HyStream 5 kDa | TangenX Pro PD HyStream 5 kDa |
| Membrane area (cm²) | 100 | 100 | 100 |
| Diafiltration buffer | 7 mM Na/K-phosphate, 0.15 M NaCl, pH 7.2 | 10 mM Tris-HCl, 150 mM NaCl pH 7.2 | 10 mM L-Histidine-HCl, 300 mM sorbitol, pH 6.2 |
| Diafiltration factor | 10 | 12.5 | 10 |
| Cross flow rate (mL/min) | 20 | 20 | 20 |
| pFeed / pRetentate / TMP (bar) | 0.4 / 0.2 - 0.3 / 0.3 | 0.4 / 0.2 - 0.3 / 0.3 | 0.4 - 0.5 / 0.2 - 0.3 / 0.3 - 0.4 |
| Permeate flux (L/h/m²) | 8.3 | 8.5 | 6.2 |
| Membrane load (mg/cm²) | 0.53 | 0.70 | 0.72 |
| Process time | 2 h 20 min | 3 h 1 min | 3 h 24 min |
| Start / end volume (ml) | 19 / 15 | 20 / 26 | 20 / 19 |
| Analysis (UV280) | Start 2.8 mg/ml End 3.6 mg/ml | Start 3.5 mg/ml End 2.8 mg/ml | Start 3.6 mg/ml End 2.9 mg/ml |
| Process yield | 102 % | 104 % | 74 % |

In all runs the retentate turned slightly cloudy by end of process. The process yield was good in the first two runs despite the retentate cloudiness. In run 3 (histidine-sorbitol buffer) the yield was poor, only 74%.

### TFF test 4 (hollow fiber)

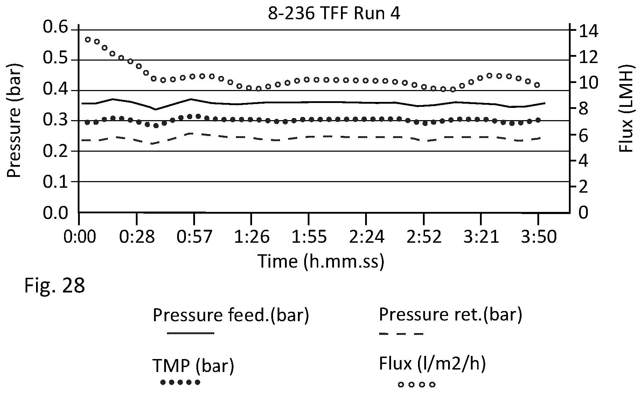

Based on TFF tests 1 - 3 it was decided to test TFF with milder conditions (less shear force). TFF test 4 was performed with a hollow fiber at a flow rate of 20 ml/min (shear rate < 2000 sec⁻¹) (Fig. 28), experiment conditions are summarized in table 29. TFF run 4 retentate appeared less cloudy than in previous runs, but process yield was only 39%. In conclusion, hollow fiber was worst of the tested TFF conditions.

**Table 29: Conditions TFF test 4.**

| | TFF Run 4 |
|---|---|
| Start material | GBS-NN Capto Adhere run 8 (1B2 - 1C4) |
| Membrane | Start AXM UFP-10-C2U, 10 kDa, 50 cm² |
| Membrane area (cm²) | 50 |
| Diafiltration buffer | 7 mM Na/K-phosphate, 0.15 M NaCl, pH 7.2 |
| Diafiltration factor | 10 |
| Cross flow rate (mL/min) | 20 |
| pFeed / pRetentate / TMP (bar) | 0.4 / 0.2 - 0.3 / 0.3 |
| Permeate flux (L/h/m²) | 10.6 |
| Process time | 3 h 53 min |
| Start / end volume (ml) | 20 / 22 |
| Analysis (UV280) | Start 3.6 mg/ml, End 1.3 mg/ml |
| Process yield | 39 % |

### Desalting test with PD-10

Based on the TFF test results, it was decided to test desalting instead. The first test was done using a PD-10 gravity flow column packed with Sephadex G-25 Medium resin. One PD-10 column was first cleaned with 1 M NaOH, then washed and equilibrated to PBS (7 mM Na/K-phosphate, 150 mM NaCl, pH 7.2). Two cycles were run, where 2.5 ml of Capto Adhere run 8 pool (1B2 - 1 C4) was loaded per cycle, allowed to enter the bed, and finally eluted with 3.5 ml PBS. The column was re-equilibrated between cycles.

The starting material and eluates of both runs were completely clear, no particles or turbidity was observed. Based on UV analysis the yield of the process after filtration of eluate was 90%.

### Desalting with HiPrep 26/10 Desalting

After successful PD-10 desalting test, it was decided to develop desalting further using pre-packed Desalting column from GE Healthcare. The goal was to determine a suitable load amount and flow rate to obtain acceptable sample dilution factor and good sample separation. Desalting experiments Run 1 and Run 2 were conducted using pre-packed desalting columns from GE Healthcare according to the experiment conditions specified in table 30 (Run 1) and table 31 (run 2)

**Table 30: Conditions HiPrep 26/10 run 1: Load test.**

| | | | |
|---|---|---|---|
| Column | HiPrep 26/10 Desalting, CV = 53 mL | Flow | 200 cm/h (17.7 ml/min) |
| Buffer | PBS (7 mM Na/K-phosphate, 0.15 M NaCl, pH 7.2) | | |
| Sample load | GBS-NN Capto Adhere Run 9 filtered pool (1B2 - 1C4), 11 ml (20% of CV) loaded. | | |
| Column cleaning and equilibration | Cleaning: 2 CV MQ, 2 CV 0.2 M NaOH, 2 CV MQ | | |
| | Equilibration: 5 CV PBS | | |
| Sample injection and elution | Equilibration: 0.5 CV | | |
| | PBS Sample load: 11 | | |
| | ml | | |
| | Elution: 1.5 CV PBS, 3 ml fractions collected | | |
| Pooling | Fractions 1A1 - 1A5 (15 ml) pooled. Filtered 0.22 µm before storage +2 - +8°C. | | |
| Sampling | No sampling | | |
| Analysis results | UV280: Start 3.0 mg/ml, filtered pool 2.0 mg/ml, fraction 1B1 0.03 mg/ml. Calculated yield 93 %. Sample dilution factor 1.47. | | |

**Table 31: Conditions HiPrep 26/10 run 2: Load test**

| | | | |
|---|---|---|---|
| Column | HiPrep 26/10 Desalting, CV = 53 ml | Flow | 200 cm/h (17.7 ml/min) |
| Buffer | PBS (7 mM Na/K-phosphate, 0.15 M NaCl, pH 7.2) | | |
| Sample load | GBS-NN Capto Adhere Run 9 filtered pool (1B2 - 1C4), 16 ml (30% of CV) loaded. | | |
| Column cleaning and equilibration | No separate cleaning or equilibration after Run 1 | | |
| Sample injection and elution | Equilibration: 0.5 CV | | |
| | PBS Sample load: 16 | | |
| | mL | | |
| | Elution: 1.5 CV PBS, 3 ml fractions collected | | |
| Pooling | Fractions 1A1 - 1B2 (21 ml) pooled. Filtered 0.22 µm before storage +2 - +8°C. | | |

| Sampling | No sampling | | |
|---|---|---|---|
| Analysis results | UV280: Start 3.0 mg/ml, filtered pool 2.1 mg/ml, fraction 1B3 0.03 mg/ml. Calculated yield 92 %. Sample dilution factor 1.43. | | |

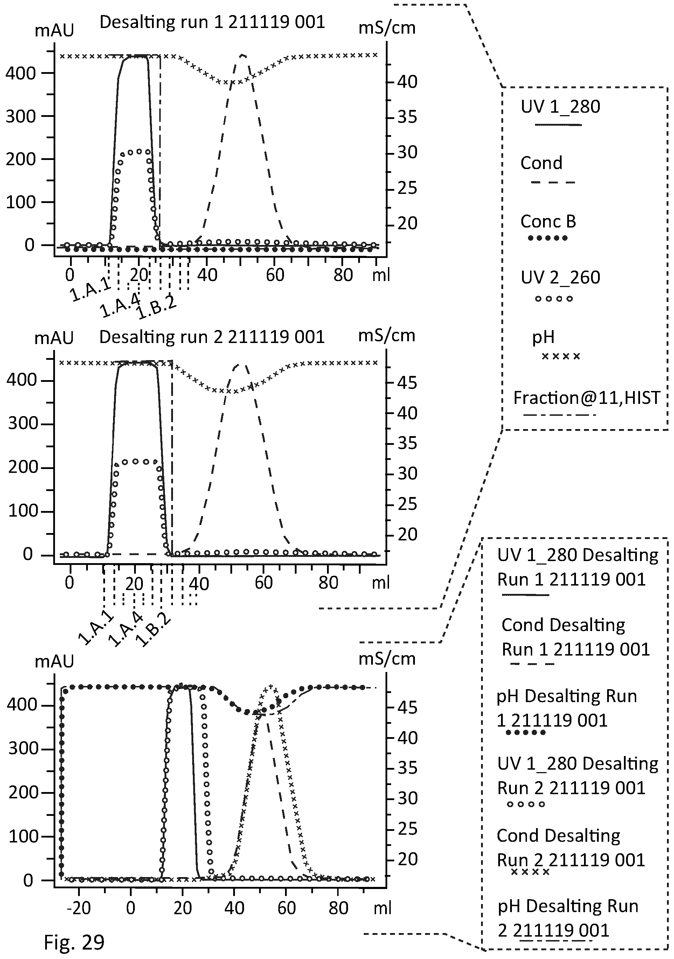

The results of the chromatograms from desalting experiment Run 1 and desalting experiment Run 2 using pre-packed desalting columns from GE Healthcare are shown in Fig. 29 (Run 1, top. Run 2, middle. Overlay of Run 1 and Run 2, bottom). In summary, desalting runs 1 and 2 were performed successfully, and sample was clear before and after process. Sample dilution factor was moderate (1.4 - 1.5) and yield acceptable (92 - 93 % after filtration). Buffer exchange performance (separation) was good, although with the higher 30% load the salt peak extends very close to product peak. With the used flow rate (200 cm/h) the recommended maximum load is approximately 25% of CV.

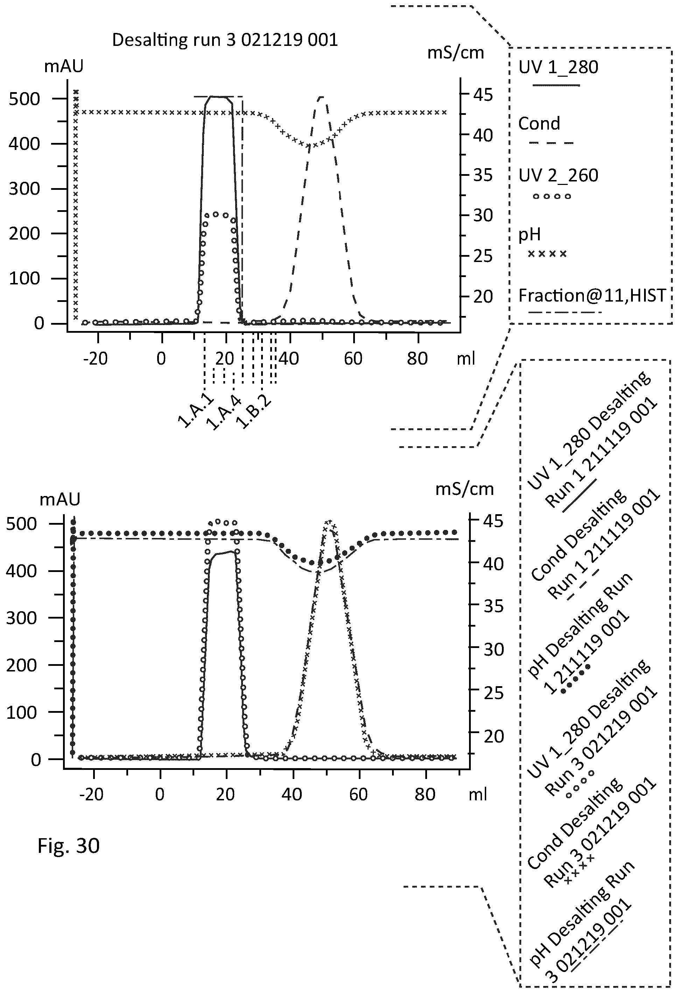

The above experiment was repeated with modified flow rate, experiment conditions are shown in table 32. The result of the experiment is shown in the chromatogram in Fig. 30 (top) and an overlay comparison of desalting experiment Run 1 and Run 4 is shown in Fig. 30 (bottom). This desalting experiment (Run 3) with lower flow rate (100 cm/h) shows that the lower flow rate appears to slightly decrease sample dilution, although this effect may rather be due to higher start material concentration. Sample yield was approximately equal to other tested conditions.

**Table 32: Conditions HiPrep 26/10 run 3: flow rate test.**

| | | | |
|---|---|---|---|
| Column | HiPrep 26/10 Desalting, CV = 53 mL | Flow | 100 cm/h (8.85 ml/min) |
| Buffer | PBS (7 mM Na/K-phosphate, 0.15 M NaCl, pH 7.2) | | |
| Sample load | GBS-NN Capto Adhere Run 8 filtered pool (1B2 - 1C4), 11 ml (20% of CV) loaded. | | |
| Column equilibration | 2 CV PBS | | |
| Sample injection and elution | Equilibration: 0.5 CV | | |
| | PBS Sample load: 11 | | |
| | mL | | |
| | Elution: 1.5 CV PBS, 3 ml fractions collected | | |
| Pooling | Fractions 1A1 - 1A5 (15 ml) pooled. Filtered 0.22 µm before storage +2 - +8°C. | | |
| Sampling | No sampling | | |
| Analysis results | UV280: Start 3.5 mg/ml, pool 2.3 mg/ml, filtered pool 2.3 mg/ml, fraction 1B1 0.06 mg/ml. Calculated yield 91 %. Sample dilution factor 1.36. | | |

In the next desalting experiment, Desalting Run 4, two HiPrep columns were used in series to mimic large scale column geometry (bed height 20 cm). Flow rate was adjusted to 150 cm/h due to increased pressure from double bed height. Load volume was 25% of CV, the suggested maximum load volume. Experiment conditions are summarized in table 33.

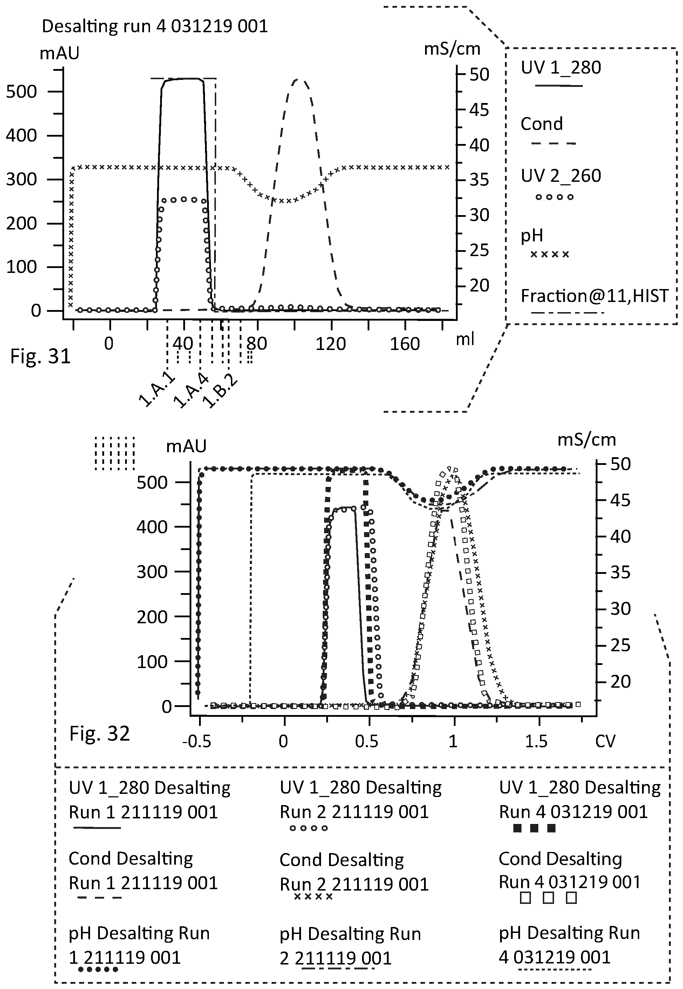

This larger setup performed slightly better (Fig. 31)(better yield, lower sample dilution) than the single HiPrep column tests. Altogether the peak shape, peak separation and elution parameters were consistent in all desalting runs despite changes in column geometry, load concentration, load amount and flow rate, as can be seen when comparing the chromatograms of different runs (see Fig. 32).

**Table 33: Conditions HiPrep 26/10 run 4: column dimension test.**

| | | | |
|---|---|---|---|
| Column | 2x HiPrep 26/10 Desalting, CV = 2x 53 mL | Flow | 150 cm/h (13.3 ml/min) |
| Buffer | PBS (7 mM Na/K-phosphate, 0.15 M NaCl, pH 7.2) | | |
| Sample load | GBS-NN Capto Adhere Run 10 filtered pool (1B2 - 1C4), 27 ml (25% of CV) loaded. | | |
| Column equilibration | 2 CV PBS | | |
| Sample injection and elution | Equilibration: 0.2 CV | | |
| | PBS Sample load: 27 | | |
| | mL | | |
| | Elution: 1.5 CV PBS, 6 ml fractions collected | | |
| Pooling | Fractions 1A1 - 1B1 (33 ml) pooled. Filtered 0.22 µm before storage +2 - +8°C. | | |
| Sampling | No sampling | | |
| Analysis results | UV280: Start 3.5 mg/ml, pool 2.8 mg/ml, filtered pool 2.8 mg/ml, fraction 1B2 0.03 mg/ml. Calculated yield 97 %. Sample dilution factor 1.24. | | |

### Analysis results of Desalting Run 4

An array of analyses were performed on Desalting Run 4 material. The material was stored at +2 - +8°C before sampling, but was after sampling transferred to storage at -15--25 °C. The analysis results are listed below in Table 34.

**Table 34. Analysis results of GBS-NN Desalting Run 4 material and comparison to GMP batch.**

| Analysis | Sample volume | Analysis performed by/ SOP | Analysis ID (Desalting Run 4) | Result BX1015300 desalting Run 4 | Result GMP batch BX1017067 DS |
|---|---|---|---|---|---|
| Appearance | 1 ml | SOP 0078 | 200526QC | Clear and colourless solution | Clear and colourless solution |
| pH | 0.5 ml | SOP 0066 | 200527QC | 7.3 | 7.1 |
| IEF | 0.2 ml | SOP 0503 | 200363AD | Comparable to reference, pl 4.6 (see appendix 4) | pl 4.6 |
| SDS-PAGE reduced | 0.2 ml | SOP 0483 | 200330AD | Comparable to reference, 38 kDa (see appendix 3) | Comparable to reference, 38 kDa |
| SDS-PAGE non-reduced | | SOP 0483 | 200329AD | >95%, main band 36 kDa (see appendix 3) | >95% |
| ELISA | 0.5 ml | SOP 0530 | 200368AD | 131 % 52 % - 330 % (95 % confidence interval) (see appendix 7) | 98 % 47 % - 203 % (95 % confidence interval) |
| SE-HPLC | 0.2 ml | SOP 0494 | 200419AD | 98.7 %, HMW peaks: 1.3 % (see appendix 5) | 98.1 % |
| RP-HPLC | 0.2 ml | SOP 0492 | 200414AD | 99.0 % (see appendix 6) | 99.0 % |
| Concentration by UV280 | 0.5 ml | SOP 0517 | 200306AD | 2.59 mg/ml | 1.75 mg/ml |
| HCP | 0.5 ml | SOP 0495 | 200386AD | 113 ng/mg (1) | 10.48 ng/mg |
| Host cell DNA by qPCR | 1.0 ml | BioOutsource / TSOP003 | 200098CQ | Below detection limit of assay (<3.125 pg/ml E. coli DNA), equating to <1.21 pg/mg | Below detection limit of assay (< 3.125 pg/ml E. coli DNA), equating to < 1.756 pg/mg |

| | | | | | |
|---|---|---|---|---|---|
| (1) result is dependent on sample dilution. | | | | | |

### Conclusions from examples 4-8

Based on the initial polishing step chromatography tests the overall differences in performance between the tested resins were small (acceptable yield and purity with all resins). The Capto Adhere resin was selected for further development and optimization, though.

For GBS-NN, the optimized Capto Adhere method consists of sample dilution (1/8) and filtration, a sample load of ≤ 16 mg/ml resin, chromatography run at 300 cm/h flow rate with 4 CV wash after load, and a 3-step elution (17 %, 55 % and 100 % buffer B). GBS-NN is eluted at the 55 % B step, with possible high-MW contaminants co-eluting in the tail of the peak. The method yield is good (>90 %) when sample load is maximum 16 mg/ml resin. Capto Adhere is also suitable for GBS-NN2 purification, but the elution parameters will need to be optimized separately.

The Capto Adhere step uses Bis-Tris/NaCl buffers (identical to process Step 1 AIEX), which necessitates a final buffer exchange step for product formulation. Both tangential flow filtration (TFF) and desalting chromatography were tested. In TFF tests two membrane types (cassette, hollow fiber) and three different buffers were tested. Although an excellent yield was obtained using a TFF cassette and either Tris or phosphate buffer, the protein solution turned slightly cloudy by end of TFF process. The gentler method of desalting chromatography was therefore chosen. The optimized desalting method consists of sample load (20 - 25 % of CV) and elution at 150 cm/h flow rate with PBS buffer, with estimated yield of >95 % and sample dilution factor 1.2 - 1.3.

Analysis results of GBS-NN material obtained from the optimized Desalting chromatography (Run 4) suggest that replacing Step 3 SEC chromatography with multimodal chromatography (Capto Adhere multimodal chromatography) and desalting chromatography yields material with comparable purity. The HCP ELISA result was higher for the Desalting Run 4 material than for GMP batch BX1017067 Drug Substance (113 ng/mg vs. 10 ng/mg), but it should be noted that here the obtained result depended on sample dilution.

In conclusion, the developed combination of multimodal chromatography and desalting chromatography is considered a feasible alternative for size exclusion chromatography for purification of recombinant fusion proteins such as GBS-NN and GBS-NN2. When following the optimized purification process disclosed above it is possible to obtain approximately 1.7 g of purified GBS-NN from 1 L starting material.

It is to be understood that while the technology proposed herein has been described in conjunction with the detailed description thereof, the foregoing description is intended to illustrate and not limit the scope of the invention, which is defined by the scope of the appended claims. Other aspects, advantages, and modifications are within the scope of the following claims.

Unless expressly described to the contrary, each of the preferred features described herein can be used in combination with any and all of the other herein described preferred features.

## Claims

1. A chromatographic method for purifying a homogenate comprising a recombinant fusion protein comprising or consisting of a first amino acid sequence having at least 90% sequence identity with the amino acid sequence of the N-terminal region of a first group B *Streptococcus* surface protein, said first amino acid sequence being fused to, optionally via linker, a second amino acid sequence having at least 90% sequence identity with the amino acid sequence of the N-terminal region of a second group B *Streptococcus* surface protein, said method comprising the steps of:
i) passing the homogenate through an anion exchange column configured to retain charged contaminants,
ii) optionally passing the homogenate obtained in step i) through a hydrophobic interaction column configured to retain hydrophobic contaminants,
iii) passing the homogenate obtained in step i) or ii) through a mixed mode column to polish the homogenate,
iv) exchanging the buffer of the homogenate obtained in step iii) to a final buffer using a desalting column, and
v) passing the homogenate obtained in step iv) through a membrane filter, to obtain the purified recombinant fusion protein in the final buffer.

2. The method according to claim 1 wherein step i) is performed using an anion exchange column comprising a diethylaminoethanol crosslinked beaded agarose resin.

3. The method according to claim 1 or 2, wherein step ii) is performed using a hydrophobic interaction column comprising a crosslinked beaded agarose resin, such as a phenyl sepharose resin.

4. The method according to any one of the preceding claims, wherein in step iii) a mixed mode column comprising a resin configured to retain DNA, host cell proteins (HCP), leached protein A, dimers and larger aggregates, and viruses, such as an agarose matrix with a multimodal N-benzyl-N-methyl ethanolamine ligand, is used.

5. The method according to any one of the preceding claims, wherein the homogenate of step ii) is diluted from 3 times to 10 times, such as from 4 to 9 times, such as from 5 to 8 times before step iii) is performed.

6. The method according to any one of the preceding claims, wherein the sample load in step iii) is from 1 g/L to 30 g/L, such as from 1 g/L to 24 g/L, such as from 1 g/L to 16 g/L (g/L column volume).

7. The method according to any one of the preceding claims, wherein step iv) is performed using a desalting column comprising a dextran resin, such as an epichlorohydrin crosslinked dextran resin, such as a Sephadex G25 resin.

8. The chromatographic method according to any one of the preceding claims, wherein each of said first and said second group B *Streptococcus* surface protein is selected from the group consisting of Rib protein, Alp1 protein, Alp2 protein, Alp3 protein, Alp4 protein and AlpC protein, with the proviso that said first and second group B *Streptococcus* surface protein are not the same.

9. The chromatographic method according to any one of the preceding claims, wherein said recombinant fusion protein is a fusion protein comprising or consisting of the N-terminal regions of the Group B *Streptococcus* Rib and AlpC proteins or the N-terminal regions of the Group B *Streptococcus* Alp1 and Alp2 proteins.

10. The chromatographic method according to any one of the preceding claims, wherein said recombinant fusion protein consists of:
i) a first amino acid sequence part consisting of 170 to 178 amino acids, preferably 174 to 175 amino acids, and having at least 90% sequence identity with the amino acid sequence shown in SEQ ID NO: 1;
ii) a second amino acid sequence part consisting of 165 to 174 amino acids, preferably 169 to 170 amino acids, and having at least 90% sequence identity with the amino acid sequence shown in SEQ ID NO: 2; and optionally:
iii) a linker amino acid sequence part consisting of 1 to 20 amino acids and separating the first amino acid sequence part from the second amino acid part, and wherein the fusion protein preferably consists of 335 to 372 amino acids, preferably 343 to 353 amino acids, more preferably 343 to 347 amino acids.

11. The chromatographic method according to any one of the preceding claims, wherein said recombinant fusion protein has an amino acid sequence of SEQ ID NO: 3 or an amino acid sequence having at least 90%, at least 92%, at least 94%, at least 96%, at least 98%, at least 99%, or 100% identity thereto.

12. The chromatographic method according to any one of claims 1-9, wherein the recombinant fusion protein consists of:
i) a first amino acid sequence part consisting of 168 to 177 amino acids, preferably 172 to 173 amino acids, and having at least 90% sequence identity with the amino acid sequence shown in SEQ ID NO: 4;
ii) a second amino acid sequence part consisting of 168 to 178 amino acids, preferably 172 to 173 amino acids, and having at least 90% sequence identity with the amino acid sequence shown in SEQ ID NO: 5; and optionally
iii) a linker amino acid sequence part consisting of 1 to 20 amino acids and separating the first amino acid sequence part from the second amino acid part,
and wherein the fusion protein preferably consists of 335 to 372 amino acids, preferably 343 to 353 amino acids, more preferably 343 to 347 amino acids.

13. The chromatographic method according to any one of claims 1-9 and 12, wherein said recombinant fusion protein has an amino acid sequence of SEQ ID NO: 6 or an amino acid sequence having at least 90%, at least 92%, at least 94%, at least 96%, at least 98%, at least 99%, or 100% identity thereto.

14. A system comprising or consisting of:
i) an anion exchange column configured to retain charged contaminants,
ii) optionally a hydrophobic interaction column configured to retain hydrophobic contaminants,
iii) a mixed mode column configured to polish the homogenate,
iv) a desalting column for buffer exchange, and
v) a membrane filter, such as a 0.2 µm filter.
